(19)

Europäisches Patentamt

European Patent Office

Office européen des brevets

(11) **EP 3 636 782 A1**

(12) **EUROPEAN PATENT APPLICATION**

(43) Date of publication:
**15.04.2020 Bulletin 2020/16**

(51) Int Cl.:
*C12R 1/225* (2006.01)   *A61K 35/747* (2015.01)

(21) Application number: **19202134.3**

(22) Date of filing: **09.10.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.10.2018   GB 201816425**

(71) Applicant: **Tervisetehnoloogiate Arenduskeskus
AS
50410 Tartu (EE)**

(72) Inventors:
• **MÄNDAR, Reet**
  **50410 Tartu (EE)**
• **HÜTT, Pirje**
  **50410 Tartu (EE)**
• **SMIDT, Imbi**
  **50410 Tartu (EE)**
• **T EPETOVA, Jelena**
  **50410 Tartu (EE)**
• **LAPP, Eleri**
  **50410 Tartu (EE)**

• **ROSTOK, Mariheleen**
  **50410 Tartu (EE)**
• **SOERUNURK, Gertu**
  **50410 Tartu (EE)**
• **RÖÖP, Tiiu**
  **50410 Tartu (EE)**
• **HOIDMETS, Dagmar**
  **50410 Tartu (EE)**
• **TAMM, Hardi**
  **50410 Tartu (EE)**
• **KOLK, Marit**
  **50410 Tartu (EE)**
• **PIISKOP, Sander**
  **50410 Tartu (EE)**
• **TAMMISTE, Triin**
  **50410 Tartu (EE)**
• **KOORT, Kairi**
  **50410 Tartu (EE)**
• **SALUMETS, Andres**
  **50410 Tartu (EE)**

(74) Representative: **Dehns
St. Bride's House
10 Salisbury Square
London EC4Y 8JD (GB)**

(54) **LACTOBACILLUS CRISPATUS PROBIOTICS**

(57)     The invention provides a Lactobacillus crispatus strain having the identifying characteristics of the strain deposited under Accession Number DSM 32717, DSM 32715, DSM 32716, DSM 32718, DSM 32719, or DSM 32720; or a mutant or variant of any thereof, a composition comprising, consisting essentially, or consisting of exactly or at least 1, 2, 3, 4, 5 or 6 different Lactobacillus crispatus strains selected from these strains, hygiene products comprising said strains, and the use of such strains and compositions in therapy.

Fig.1

EP 3 636 782 A1

**Description**

Field of invention

The present invention relates to novel bacterial strains, bacterial consortia, and the use thereof as probiotics for nutritional and/or therapeutic applications.

Background

Vaginitis is inflammation of the vagina that can cause different complaints, including itching, discomfort and discharge. In addition, vaginitis may facilitate sexually transmitted infections and may be related to reproductive failure. Vaginal discharge is one of the most common reasons for a gynecological consultation. Bacterial vaginosis (BV) and vulvovaginal candidiasis (WC) are the main causes of discharge, and vaginitis more generally. Bacterial vaginosis (BV) is an imbalance of the vaginal microbiota. In the clinical setting, BV may present asymptomatically, but it is often accompanied by an increased pH, thin vaginal discharge, fishy odour, and the presence of vaginal epithelial cells covered in bacteria. The gold standard for laboratory-based BV diagnosis is the Nugent scoring method - the higher the score, the higher the likelihood of BV being present.

VVC is typically caused by the yeast *Candida albicans* or some other *Candida* species. BV and WC are fairly prevalent in the general population, have a chronic and/or recurrent nature and are associated with different complications and a poor quality of life. The mainstream antimicrobial treatment is not always effective, and problems remain due to bacterial and yeast resistance and side effects. Therefore alternative treatments are of interest to patients and their health care providers.

In addition, recurrent urinary tract infections (UTIs) are a common clinical problem across the lifespan of women, the majority of cases being caused by *Escherichia coli*. The status of vaginal microbiota plays a key role in the pathogenesis of UTI: alterations in the vaginal microbiota are associated with risks for UTI, and effects on the vaginal microbiota associated with treatment of UTIs can affect the success of this therapy.

There is interest in the potential use of probiotics to treat such conditions. However, to be effective, probiotics must survive manufacturing processing, packaging and storage conditions. Moreover, when desired for oral administration, the probiotics must survive transit through the gastrointestinal tract to have a positive health effect.

There remains a need for new treatments for conditions such as BV and WC.

Disclosure of invention

The present inventors have identified and characterised new strains of Lactobacillus crispatus that have a beneficial effect on the vaginal microbiota. The strains were deposited by the applicant with the Leibniz Institute DSMZ-German Collection of Microorganisms and Cell Cultures (Inhoffenstrasse 7 B, D-38124 Braunschweig, Germany) on 14 December 2017; and were given the following Accession Numbers: DSM 32715 (internal designation EST6), DSM 32716 (internal designation EST1), DSM 32717 (internal designation EST2), DSM 32718 (internal designation EST3), DSM 32719 (internal designation EST4), DSM 32720 (internal designation EST5).

These strains were isolated from the vaginal tract of healthy women, indicating that they may be considered to have GRAS (generally recognized as safe) status, i.e. that they are suitable for oral and/or vaginal consumption/administration.

Characteristics of these strains are shown in the Examples and summarised below. Thus, the strain may have characteristics selected from the following, e.g. it may have most or all of these characteristics

(a) the ability to produce lactic acid;

(b) the ability to produce $H_2O_2$;

(c) the ability to metabolise D-galactose, D-glucose, D-fructose, D-mannose, D-maltose, D-lactose, and D- saccharose;

(d) the inability to metabolise Glycerol, Erythritol, D-arabinose, L-arabinose, D-ribose, D-xylose, L-xylose, D-adonitol, Methyl-βD-Xylopyranoside, L-sorbose, L-rhamnose, Dulcitol, Inositol, D-sorbitol, Methyl-αD-Mannopyranoside, amygdalin, arbutin, esculin ferric citrate, D-cellobiose, D-Melibiose, D-Trehalose, Inulin, D-Melezitose, D-Raffinose, Xylitol, Gentiobiose, D-Turanose, D-Lyxose, D-Tagatose, D-Fucose, L-Fucose, D-Arabitol, L-Arabitol, potassium Gluconate, potassium 2-ketogluconate and potassium 5-ketogluconate;

(e) susceptibility to ampicillin, chloramphenicol, clindamycin, erythromycin, gentamicin, nitrofurantoin, tetracycline, and vancomycin;

(f) possess no transferrable antibiotic resistance;

(g) possess no significant haemolytic activity;

(h) form little or no significant autoaggregation;

(i) have cell-adhesive properties, in particular have the ability to adhere to HeLa cells;

(j) have antagonistic activity against a urophathogenic bacterium, particularly against *E. coli ATCC* 700414, *E. coli ATCC* 700336 and/or *E. coli* strains isolated from women with bacterial vaginosis, such as *E. coli* RTN038, *E. coli* RTN047, and/or E.coli RTN100;

(k) have antagonistic activity against *Candida spp,* particularly against *Candida albicans* ATCC 32032, *Candida albicans* RTN 071, and/or *Candida glabrata* RTN 009;

(l) have antagonistic activity against *Gardnerella vaginalis,* particularly *Gardnerella vaginalis* ATCC 14018 (DSM 4944); and/or

(m) have the ability to express proteins associated with probiotic activity, e.g. one or more proteins shown in **Table 8**, particularly a bacteriocin and/or bacteriocin transporter.

Methods for determining any of these abilities are known in the art and described in the Examples.

Thus, in one aspect there is provided a *Lactobacillus crispatus* strain having the identifying characteristics of the strain deposited under Accession Number DSM 32715, DSM 32716, DSM 32717, DSM 32718, DSM 32719, or DSM 32720. The identifying characteristics are characteristics (a) to (m) above. Preferably, the strain has at least 4, 5, 6, 7, 8, 9, 10, 11, 12 or all of these characteristics.

Preferably, the *Lactobacillus crispatus* strain has at least characteristics (f) and (g). Preferably, the *Lactobacillus crispatus* strain has at least characteristics (f), (g) and one or more of characteristics (j), (k) and (l). Preferably, the *Lactobacillus crispatus* strain further has characteristic (i). Thus, preferably, the *Lactobacillus crispatus* strain has at least characteristics (f), (g) and (i) and one or more of characteristics (j), (k) and (l).

Also provided is a mutant or variant of such a strain. The mutant or variant should at least substantially retain some, most, or all of the characteristics (a) to (m) listed above of the wild-type strain, e.g. at least 4, 5, 6, 7, 8, 9, 10, 11, 12 or all of these characteristics. Thus, it may, e.g., differ from the wild-type strain only in the features specifically recited to characterise the mutant or variant.

For example, provided is a mutant or variant of a *Lactobacillus crispatus* strain having the identifying characteristics of the strain deposited under Accession Number DSM 32715, DSM 32716, DSM 32717, DSM 32718, DSM 32719, or DSM 32720, characterised in that it contains a recombinant gene or vector.

The recombinant gene or vector may, e.g., encode a protein that may enhance the probiotic effectiveness of the bacterium. For example, it may encode a bacteriocin, a bacteriocin transporter, a lysin a protein involved in adhesion and/or aggregation, or a combination of any thereof.

Any reference herein to a "Lactobacillus crispatus strain provided herein" or simply a "strain provided herein" should be understood to encompass a Lactobacillus crispatus strain having the identifying characteristics of the strain deposited under Accession Number DSM 32715, DSM 32716, DSM 32717, DSM 32718, DSM 32719, or DSM 32720 or a mutant or variant thereof as defined above; a Lactobacillus crispatus strain having the identifying characteristics of the strain deposited under Accession Number DSM 32715, DSM 32716, DSM 32717, DSM 32718, DSM 32719, or DSM 32720 is preferred.

Preferably, the Lactobacillus crispatus strain of the invention has the identifying characteristics of the strain deposited under Accession Number DSM 32715. Preferably, the Lactobacillus crispatus strain of the invention has the identifying characteristics of the strain deposited under Accession Number DSM 32716. Preferably, the Lactobacillus crispatus strain of the invention has the identifying characteristics of the strain deposited under Accession Number DSM 32717. Preferably, the Lactobacillus crispatus strain of the invention has the identifying characteristics of the strain deposited under Accession Number DSM 32718. Preferably, the Lactobacillus crispatus strain of the invention has the identifying characteristics of the strain deposited under Accession Number DSM 32719. Most preferably, the Lactobacillus crispatus strain of the invention has the identifying characteristics of the strain deposited under Accession Number or DSM 32720.

Reference is made throughout to Lactobacillus crispatus strains having the identifying characteristics of the strain deposited under Accession Number DSM 32715, DSM 32716, DSM 32717, DSM 32718, DSM 32719, or DSM 32720. The preferred strains having such characteristics of the strain deposited under Accession Number DSM 32715, DSM 32716, DSM 32717, DSM 32718, DSM 32719 or DSM 32720 are the strains themselves deposited under Accession Number DSM 32715, DSM 32716, DSM 32717, DSM 32718, DSM 32719 or DSM 32720.

Thus, preferably the strain of the invention is the strain deposited under Accession Number DSM 32715, DSM 32716, DSM 32717, DSM 32718, DSM 32719 or DSM 32720. Most preferably, the strain is that deposited under Accession number DSM 32720.

Each Lactobacillus crispatus strain provided herein may be a "biologically pure" strain. Also provided is a biologically pure culture of exactly or at least 1 or more of any of the strains provided herein. By "biologically pure" is meant that no, or substantially no, other microbial strain other than the specified strain(s) is present.

The strains provided herein may be considered to be "isolated", i.e. removed from their natural environment.

The Lactobacillus crispatus strain provided herein may be a culture in liquid form, frozen form, dried form (e.g. spray-dried) or freeze-dried (lyophilised) form. The Examples show that the freeze-dried form has an excellent shelf life, with viability remaining substantially unchanged during storage at -20° C for one year. Thus, the freeze-dried form, e.g. a lyophilised powder, is of particular interest.

Also provided is a composition comprising, consisting essentially, or consisting of mixtures or combinations of probiotic bacteria. In particular, provided is a composition comprising, consisting essentially, or consisting of exactly or at least 1, 2, 3, 4, 5 or 6 different Lactobacillus crispatus strains selected from those having the identifying characteristics of the strains deposited under Accession Number DSM 32715, DSM 32716, DSM 32717, DSM 32718, DSM 32719, and/or DSM 32720.

Preferably, the composition comprises at least or exactly 2, or at least or exactly 3 different Lactobacillus crispatus strains selected from those having the identifying characteristics of the strains deposited under Accession Number DSM 32715, DSM 32716, DSM 32717, DSM 32718, DSM 32719, and/or DSM 32720.

Each of the strains DSM 32715, DSM 32716, DSM 32717, DSM 32718, DSM 32719, and DSM 32720 has been demonstrated in the Examples of having advantageous properties in relation to, and/or effects on, the vaginal microbiota. Any combination of these strains is encompassed within the present invention.

Preferably, the compositions of the invention comprise one of the following combinations of strains:

| Combination # | Strains present in combination (reference to a deposited strain is reference to a strain having the identifying characteristics of the deposited strain, or a mutant or variant thereof) |
|---|---|
| 1 | DSM 32715 and DSM 32716 |
| 2 | DSM 32715 and DSM 32717 |
| 3 | DSM 32715 and DSM 32718 |
| 4 | DSM 32715 and DSM 32719 |
| 5 | DSM 32715 and DSM 32720 |
| 6 | DSM 32716 and DSM 32717 |
| 7 | DSM 32716 and DSM 32718 |
| 8 | DSM 32716 and DSM 32719 |
| 9 | DSM 32716 and DSM 32720 |
| 10 | DSM 32717 and DSM 32718 |
| 11 | DSM 32717 and DSM 32719 |
| 12 | DSM 32717 and DSM 32720 |
| 13 | DSM 32718 and DSM 32719 |
| 14 | DSM 32718 and DSM 32720 |
| 15 | DSM 32719 and DSM 32720 |

Preferably, the compositions of the invention comprise one of the following combinations of strains:

| | Combination # | Strains present in combination (reference to a deposited strain is reference to a strain having the identifying characteristics of the deposited strain, or a mutant or variant thereof) |
|---|---|---|
| | 16 | DSM 32715, DSM 32716 and DSM 32717 |
| | 17 | DSM 32715, DSM 32716 and DSM 32718 |
| | 18 | DSM 32715, DSM 32716 and DSM 32719 |
| | 19 | DSM 32715, DSM 32716 and DSM32720 |
| | 20 | DSM 32715, DSM 32717 and DSM 32718 |
| | 21 | DSM 32715, DSM 32717 and DSM 32719 |
| | 22 | DSM 32715, DSM 32717 and DSM 32720 |
| | 23 | DSM 32715, DSM 32718 and DSM 32719 |
| | 24 | DSM 32715, DSM 32718 and DSM 32720 |
| | 25 | DSM 32715, DSM 32719 and DSM 32720 |
| | 26 | DSM 32716, DSM 32717 and DSM 32718 |
| | 27 | DSM 32716, DSM 32717 and DSM 32719 |
| | 28 | DSM 32716, DSM 32717 and DSM 32720 |
| | 29 | DSM 32716, DSM 32718 and DSM 32719 |
| | 30 | DSM 32716, DSM 32718 and DSM 32720 |
| | 31 | DSM 32716, DSM 32719 and DSM 32720 |
| | 32 | DSM 32717, DSM 32718 and DSM 32719 |
| | 33 | DSM 32717, DSM 32718 and DSM 32720 |
| | 34 | DSM 32717, DSM 32719 and DSM 32720 |
| | 35 | DSM 32718, DSM 32719 and DSM 32720 |

Preferably, the compositions of the invention comprise one of the following combinations of strains:

| | Combination # | Strains present in combination (reference to a deposited strain is reference to a strain having the identifying characteristics of the deposited strain, or a mutant or variant thereof) |
|---|---|---|
| | 36 | DSM 32715, DSM 32716, DSM 32717 and DSM 32718 |
| | 37 | DSM 32715, DSM 32716, DSM 32717 and DSM 32719 |
| | 38 | DSM 32715, DSM 32716, DSM 32717 and DSM 32720 |
| | 39 | DSM 32715, DSM 32716, DSM 32718 and DSM 32719 |
| | 40 | DSM 32715, DSM 32716, DSM 32718 and DSM 32720 |
| | 41 | DSM 32715, DSM 32716, DSM 32719 and DSM 32720 |
| | 42 | DSM 32715, DSM 32717, DSM 32718 and DSM 32719 |
| | 43 | DSM 32715, DSM 32717, DSM 32718 and DSM 32720 |
| | 44 | DSM 32715, DSM 32717, DSM 32719 and DSM 32720 |
| | 45 | DSM 32715, DSM 32718, DSM 32719 and DSM 32720 |
| | 46 | DSM 32716, DSM 32717, DSM 32718 and DSM 32719 |
| | 47 | DSM 32716, DSM 32717, DSM 32718 and DSM 32720 |
| | 48 | DSM 32716, DSM 32717, DSM 32719 and DSM 32720 |

(continued)

| Combination # | Strains present in combination (reference to a deposited strain is reference to a strain having the identifying characteristics of the deposited strain, or a mutant or variant thereof) |
|---|---|
| 49 | DSM 32716, DSM 32718, DSM 32719 and DSM 32720 |
| 50 | DSM 32717, DSM 32718, DSM 32719 and DSM 32720 |

Preferably, the compositions of the invention comprise one of the following combinations of strains:

| Combination # | Strains present in combination (reference to a deposited strain is reference to a strain having the identifying characteristics of the deposited strain, or a mutant or variant thereof) |
|---|---|
| 51 | DSM 32715, DSM 32716, DSM 32717,DSM 32718 and DSM 32719 |
| 52 | DSM 32715, DSM 32716, DSM 32717,DSM 32718 and DSM 32720 |
| 53 | DSM 32715, DSM 32716, DSM 32717,DSM 32719 and DSM 32720 |
| 54 | DSM 32715, DSM 32716, DSM 32718,DSM 32719 and DSM 32720 |
| 55 | DSM 32715, DSM 32717, DSM 32718, DSM 32719 and DSM 32720 |
| 56 | DSM 32716, DSM 32717,DSM 32718, DSM 32719 and DSM 32720 |

Preferably, the composition comprises Lactobacillus crispatus strains having the identifying characteristics of the strains deposited under Accession Number DSM 32715 DSM 32716, DSM 32717,DSM 32718, DSM 32719 and DSM 32720, or a mutant or variant thereof (combination #57).

In particular, the composition may comprise or consist of at least a Lactobacillus crispatus strain having the identifying characteristics of the strain deposited under Accession Number DSM 32717, DSM 32718, or DSM 32720, or a combination of any 2 or all 3 thereof.

Alternatively viewed, the composition preferably comprises a combination of strains selected from the group consisting of combination #10, #12 and #14. The composition may preferably comprise a combination of strains selected from the group consisting of combination #20, #22,#24, #26, #28, #30, #32, #33, #34 and #35. Combination #33 is particularly preferred. The composition may preferably comprise a combination of strains selected from the group consisting of combination #36, #38, #40, #42, #43, #44, #45, #46, #47, #48, #49 and #50. A combination selected from the group consisting of combination #43, #47 and #50 is preferred, The composition may preferably comprise a combination of strains selected from the group consisting of combination #51, #52, #53, #54, #55 and #56. A combination selected from the group consisting of combination #52, #55 and #56 is preferred.

Particularly preferably, the composition comprises Lactobacillus crispatus strains having the identifying characteristics of the strains deposited under Accession Number DSM 32717 and DSM 32720, or a mutant or variant thereof (Combination #12). Preferably the composition comprises a combination of strains selected from the group consisting of combination #22, #28, #33 and #34. Combination #33 is particularly preferred. Preferably the composition comprises a combination of strains selected from the group consisting of combination #38, #43, #44, #47, #48 and #50, more preferably #43, #47 and #50. Preferably the composition comprises a combination of strains selected from the group consisting of combination #52, #53, #55 and #56, more preferably #52, #55 and #56. These compositions (i.e. compositions comprising Lactobacillus crispatus strains having the identifying characteristics of the strains deposited under Accession Number DSM 32717 and DSM 32720, or a mutant or variant thereof), are preferably used in the methods of treatment of the invention, particularly the treatment of bacterial vaginosis.

Particularly preferably, the composition comprises Lactobacillus crispatus strains having the identifying characteristics of the strains deposited under Accession Number DSM 32718 and DSM 32720, or a mutant or variant thereof (Combination #14). Preferably the composition comprises a combination of strains selected from the group consisting of combination #24, #30, #33 and #35. Combination #30 is particularly preferred. Preferably the composition comprises a combination of strains selected from the group consisting of combination #40, #43, #45, #47, #49 and #50, more preferably #40, #47 and #49. Preferably the composition comprises a combination of strains selected from the group consisting of combination #52, #54, #55 and #56, more preferably #52, #54 and #56. These compositions, i.e. compositions comprising Lactobacillus crispatus strains having the identifying characteristics of the strains deposited under Accession Number DSM 32718 and DSM 32720, or a mutant or variant thereof, particularly those compositions comprising Lactobacillus crispatus strains having the identifying characteristics of the strains deposited under Accession Number DSM 32716, DSM 32718 and DSM 32720, or a mutant or variant thereof are preferably used in the methods of treatment of the invention, particularly the treatment of vulvovaginal candidiasis.

Alternatively, the composition may comprise or consist of exactly or at least 2 or 3 different Lactobacillus crispatus strains selected from those having the identifying characteristics of the strains deposited under Accession Number DSM 32715, DSM 32717, and/or DSM 32720.

Alternatively, the composition may comprise or consist of exactly or at least 2 or 3 different Lactobacillus crispatus strains selected from those having the identifying characteristics of the strains deposited under Accession Number DSM 32715, DSM 32716, and/or DSM 32719.

Preferably, the composition of the invention comprises any one of the above combinations of strains. Preferably, the Lactobacillus crispatus strains comprised within the composition consist of those Lactobacillus crispatus strains of one of the above mentioned combinations, i.e. preferably the composition foes not comprise further Lactobacillus crispatus strains.

The composition may further comprise one or more further (different) strains of probiotic bacteria, which may, e.g. be selected from

(i) Lactobacillus crispatus mutants and/or variants as defined above;

(ii) different Lactobacillus crispatus strains; and/or

(iii) any other probiotic bacteria.

The term "probiotic bacteria" as used herein refers to live microorganisms which, when administered in appropriate amounts to a subject, affect the microbiota of the subject, thereby preferably conferring a health benefit on the subject.

Such "other probiotic bacteria" may, e.g., be lactic acid bacteria. They may, e.g., be from the genera Lactobacillus, Lactococcus, Bifidobacterium, Streptococcus and/or Enterococcus. They may, e.g., be from the species Lactobacillus acidophilus, L. brevis, L. bulqaricus, L. casei, L. delbrueckii, L. divergens, L. gasseri, L. helveticus, L. johnsonii, L. kunkeei, L. Lactis, L. paracasei, L. plantarum, L. reuteri, L. rhamnosus, L. sakei, L. Salivarius, Lactococcus diacetylactis Lactococcus lactis, Lactococcus thermophilus, Bifidobacterium bifidum, Bifidobacterium lactis, Bifidobacterium longum, Enterococcus faecium and/or Streptococcus thermophiles.

Many probiotic strains are available from public culture collections such as the American Type Culture Collection (ATCC), e.g., L. delbrueckii (ATCC 10705), L. acidophilus (ATCC4356), L. crispatus (ATCC 33820), L. gasseri (ATCC 9857), L.jensenii (ATCC 25258), L. paracasei (ATCC 27092), L.rhamnosus (ATCC 53103), L. fermentum (ATCC 14932), L.plantarum (ATCC 14917), L. reuteri (ATCC 5 53608) and L.salivarius (ATCC 29602).

Thus, provided are mixtures or combinations of probiotic bacteria comprising, consisting essentially, or consisting of exactly or at least 1, 2, 3, 4, 5 or 6 different Lactobacillus crispatus strains selected from those having the identifying characteristics of the strain deposited under Accession Number DSM 32715, DSM 32716, DSM 32717, DSM 32718, DSM 32719, and/or DSM 32720, which may additionally include further strains of probiotic bacteria, e.g. as defined above. Alternatively, the mixtures or combinations may include no bacteria other than Lactobacillus crispatus strains selected from those having the identifying characteristics of the strain deposited under Accession Number DSM 32715, DSM 32716, DSM 32717, DSM 32718, DSM 32719, and/or DSM 32720, i.e. the only bacteria that may be present in the mixtures or combinations must be selected from Lactobacillus crispatus strains having the identifying characteristics of the strain deposited under Accession Number DSM 32715, DSM 32716, DSM 32717, DSM 32718, DSM 32719, and/or DSM 32720. Preferred combinations are those described above, particularly combinations #1 to #57. These are also preferred mixtures of strains.

Such mixtures or combinations of probiotic bacteria may be administered together in a single composition or be administered separately. For example, they may be in a combined preparation for separate, simultaneous, or sequential use in maintaining or restoring a beneficial microbiota, e.g. a vaginal microbiota. They may be in a combined preparation for separate, simultaneous, or sequential use in therapy, e.g. the treatment or prevention of dysbiosis, vaginosis, vulval and/or vaginal yeast infection, villitis, and/or urinary tract infection.

The mixtures or combinations of probiotic bacteria may be a bacterial consortium, so the strains may have a symbiotic relationship and/or have a synergistic effect on a host subject.

The compositions, mixtures or combinations may, e.g., further comprise one or more further components, e.g. selected from (a) one or more source(s) of nutrients; (b) one or more mineral salt(s); (c) one or more cryoprotectants; (d) one or more stabilizing agent(s); and/or (e) one or more agents with antimicrobial properties.

A stabilizing agent may, e.g., be a pH stabilizing agent, e.g. selected from citric acid, Vitamin C, and/or sodium bicarbonate.

A mineral salt may, e.g., be selected from magnesium, zinc, iron, manganese, sodium, calcium and/or potassium.

A nutrient may, e.g., be selected from carbohydrates, such as monosaccharides, disaccharides and/or oligosaccharides; fats; a nitrogen source, such as ammonium, nitrates, amino acid(s), peptide(s) and/or protein(s); and/or vitamins.

A fat may, e.g., be a fatty acid, e.g. selected from omega-3 and/or omega-6 fatty acids.

A vitamin may, e.g., be selected from Vitamin A, vitamin B (such as folic acid, Vitamin B6 and/or vitamin B12), vitamin C, Vitamin D, Vitamin K2, and/or vitamin E.

A monosaccharide may, e.g. be selected from arabinose, D-glucose, D-galactose, D-mannose, ribose, tagatose, deoxyribose, glycerose; dihydroxyacetone, D-fructose, D-xylose, xylitol, and/or seduloheptulose.

A disaccharide may, e.g. be selected from xylobiose, sucrose, maltose, lactose, lactulose, trehalose, cellobiose, and combinations thereof.

A polysaccharide may, e.g. be selected from inulin, fructooligosaccharides, galactooligosaccharides, isomaltooligosaccharides, xylooligosaccharides, and/or pectic oligosaccharides.

For example, the nutrient(s) may include one or more selected from D-galactose, D-glucose, D-fructose, D-mannose, D-maltose, D-lactose, and/or D- saccharose.

The composition may optionally exclude Glycerol, Erythritol, D-arabinose, L-arabinose, D-ribose, D-xylose, L-xylose, D-adonitol, Methyl-βD-Xylopyranoside, L-sorbose, L-rhamnose, Dulcitol, Inositol, D-sorbitol, Methyl-aD-Mannopyranoside, amygdalin, arbutin, esculin ferric citrate, D-cellobiose, D-Melibiose, D-Trehalose, Inulin, D-Melezitose, D-Raffinose, Xylitol, Gentiobiose, D-Turanose, D-Lyxose, D-Tagatose, D-Fucose, L-Fucose, D-Arabitol, L-Arabitol, potassium Gluconate, potassium 2-ketogluconate and/or potassium 5-ketogluconate.

The nutrient may, in particular, be a prebiotic. By "prebiotic" is meant an agent that is only poorly digestible or non-digestible by the host, e.g. the human or animal subject, but that can be utilised as a nutrient by a target microorganism. The target microorganism may be selected from probiotic bacteria that are endogenous to the host and/or from probiotic bacteria that are administered to the host. Of particular interest is a prebiotic that is only poorly digestible or non-digestible by human subjects, but that can be utilised as a nutrient by a probiotic bacterium, particularly by one or more of the Lactobacillus crispatus strains provided herein.

Thus, the composition provided herein may comprise, consist essentially of, or consist of (i) one or more of the probiotic Lactobacillus crispatus strains provided herein; and (ii) one or more prebiotics, particularly one that can be utilised as a nutrient by said strain(s). Such a composition may have a synergistic effect.

The agent with antimicrobial properties may, e.g. be selected from an antibacterial drug, an anti-fungal drug, an anti-viral drug, and/or a natural substance with antimicrobial properties or the active extract thereof, such as garlic or allicin. Thus, it may, e.g. be selected from metronidazole, clotrimazole, terconazole, clindamycin, butoconazole, hydroxyquinoline, miconazole, povidone iodine, fluconazole, nystatin, garlic, Oil Of Oregano, Echinacea, and/or Manuka Honey or an active extract thereof.

The composition may be a pharmaceutical composition. Thus, it may comprise a pharmaceutically acceptable carrier, diluent or excipient.

The pharmaceutical compositions can be formulated according to any of the conventional methods known in the art and widely described in the literature. Thus, one or more of the Lactobacillus crispatus strains provided herein may be incorporated, optionally together with other active substances (examples of which are as described below), with one or more conventional pharmaceutically acceptable carriers, diluents and/or excipients, etc., appropriate for the particular use for a composition, to produce conventional preparations which are suitable or can be made suitable for administration.

The composition may be formulated as liquids, as semi-solids or as solids, e.g. liquid solutions, dispersions, suspensions, tablets, capsules, pills, suppositories, powders, sachets, cachets, elixirs, emulsions, syrups, creams, gels, and the like. It may be provided e.g. as a gastric fluid-resistant preparation and/or in sustained action form. It may be a form suitable for oral, topical, rectal, intravaginal, genital, and/or intranasal administration. The preferred form depends on the intended mode of administration and therapeutic application.

The preferred mode of administration is oral or vaginal, particularly oral. Any physiologically compatible carrier, excipient, diluent, buffer or stabilizer can be used in the compositions of the invention. Examples of suitable carriers, excipients, diluents, buffers and stabilizers include one or more of water, saline, phosphate buffered saline, dextrose, glycerol, ethanol, microcellulose, and the like, as well as combinations thereof. In some cases isotonic agents, e.g. sugars, polyalcohols (e.g. mannitol, sorbitol), or sodium chloride may be included. The compositions may additionally include lubricating agents, wetting agents, emulsifying agents, suspending agents, preserving agents, sweetening agents, flavouring agents, and the like. The compositions may be formulated so as to provide quick, sustained or delayed release of the active ingredient after administration to the subject by employing procedures well known in the art. An enteric coating may optionally be used.

The composition may be a nutraceutical composition. The term "nutraceutical" as used herein denotes a product that may provide health benefits in addition to nutritional benefits. Thus, it may comprise a nutrient that can be utilised by the host, i.e. the intended consumer of the composition.

The nutraceutical composition may be formulated as a supplement, e.g. it may be a solid formulation such as capsules, tablets or a powder, or a liquid formulation, such as solutions or suspensions. It may also be formulated as a food for humans, or a feed for non-human animals, by combining or incorporating it with one or more further edible materials. The edible materials may be liquid or solid.

Food products which may be formulated to comprise a nutraceutical composition provided herein include, but are not limited to, instant powder products, food bars such as candy bars or protein bars, meat and meat analogue products, fish and fish analogue products, breakfast cereals, vegetable fat spreads, mayonnaise, dairy products, soy milk products, pasta, noodles, baked goods such as bread, pastry, cakes and biscuits, ready meals, infant formula and baby food.

Suitable examples of dairy products include butter, cheese, ice cream, yogurt, whipping cream, sour cream and cottage cheese. Examples of meat products include, but are not limited to, processed meat such as pate, ham, spam, sausage and whole muscle meat. The meat analogue may be a textured vegetable, fungal or dairy protein that mimics meat in texture such as quorn or tofu.

Non-limiting examples of protein fortified liquid beverages which may be formulated to comprise nutraceutical composition provided herein include carbonated fortified soft drinks, beer, fruit juices, fruit-flavoured drinks, vegetable drinks, sports drinks, soy milk drinks, rice milk drinks, infant formula, milk, flavoured milk drinks, goat milk, liquid yogurt, buttermilk, or combinations thereof.

Typical components of food, feeds or nutraceutical compositions, in addition to the Lactobacillus crispatus strains provided herein, may include protein-bearing substances, fat-containing substances, carbohydrates, polysaccharides, starch-bearing substances, crude fibres, ash, minerals, trace elements, vitamins, fatty acids, proteins, peptides, amino acids, herbs, lipids, antioxidants, carotenoids, tocopherols, tocotrienols, phytosterols, polyphenols, bioflavonoids and/or dietary fibre. Thus, these are all examples of suitable edible materials which may be used to prepare a nutraceutical composition, food or feed.

Suitable starch-bearing substances may for example be derived from grains, e.g. selected from the group consisting of corn, soybean, wheat, sorghum, barley, oat, and mixtures thereof. Examples of suitable starch-bearing substances include, but are not limited to, corn flour, ground corn, soybean flour, wheat flour, ground oat flour, wheat middlings, soybean meal, corn grit, and mixtures thereof.

A crude protein-bearing substance such as, for example, a fish meal, dried whey, a soybean meal, and mixture thereof may be used. Other suitable protein-bearing substances include, but are not limited to, soybean protein concentrate, soy flour, blood meal, plasma protein, dried skim milk, whey protein concentrate, canola meal, corn gluten meal, wheat gluten meal, dried yeast, sunflower meal, and mixtures thereof.

Suitable fat-containing substances include, but are not limited to, lard, tallow, soybean oil, lecithin, coconut oil, whey-fat blend, and mixtures thereof.

In a further aspect, there is provided a method of preparing a nutraceutical composition comprising one or more of the Lactobacillus crispatus strains provided herein. Said method may comprise combining one or more of the Lactobacillus crispatus strains provided herein with an edible material. Suitable edible materials are discussed above.

In another aspect, there is provided a method of preparing a food, drink or feed fortified with one or more of the Lactobacillus crispatus strains provided herein. Said method may comprise combining one or more of the Lactobacillus crispatus strains provided herein with an edible material; or incorporating one or more of the Lactobacillus crispatus strains provided herein into a food, drink or feed. Suitable foods drinks and feed are discussed above.

Also provided is a hygiene product, which may be an absorbent product, e.g. selected from as a sanitary napkin, diaper, panty liner, tampon, incontinence guard, and/or hygiene tissue, said product comprising one or more of the Lactobacillus crispatus strains provided herein and/or a composition as provided herein. The Lactobacillus crispatus strain(s) and/or composition, which may preferably be in the form of a lyophilised powder, may be sprayed, soaked or otherwise applied into or onto the hygiene product, or a relevant part thereof.

For example, in the case of a sanitary napkin, the Lactobacillus crispatus strain(s) and/or composition should be applied to at least part of the side that is designed to come into contact with the subject's skin during normal use; it may, but need not be, applied to the side that is in direct contact with the undergarment during normal use.

As shown in the Examples, the Lactobacillus crispatus strains provided herein can modulate the vaginal microbiota. The Lactobacillus crispatus strains provided herein have advantageous characteristics, such as antagonistic activity against certain microbes that have an unfavourable effect on the human microbiome, particularly the vaginal microbiome.

Consequently, in a further aspect there is provided a Lactobacillus crispatus strain as provided herein and/or a composition as provided herein for use in modulating the microbiota of a subject. The "microbiota" is the population of microorganisms (i.e. microbes) that has colonised a region of the host. The microbiota may, e.g. be vaginal, urethral, oral (including nasal), intestinal, and/or skin. The vaginal microbiota is of particular interest.

Each microbiota is different, so the types and proportions of different bacteria present in the healthy vaginal microbiota differ from those present in the healthy intestinal microbiota.

"Modulating" the microbiota may include maintaining a (healthy) microbiota and/or restoring a healthy/beneficial microbiota. Thus, modulating the vaginal microbiota may include maintaining the vaginal microbiota, in particular maintaining the lactic acid, e.g. Lactobacillus proportion in the vaginal microbiota. Modulating the vaginal microbiota may include restoring a healthy/beneficial vaginal microbiota, e.g. increasing the lactic acid, e.g. Lactobacillus proportion in the vaginal microbiota and/or decreasing the proportion of dysbiotic and/or pathogenic bacteria in the vaginal microbiota.

The vaginal microbiome may be assessed via a Nugent score (Nugent, et al. 1991 "Reliability of diagnosing bacterial vaginosis is improved by a standardized method of gram stain interpretation". Journal of Clinical Microbiology. 29 (2): 297-301). A low score of 0-3 is indicative of a healthy vaginal microbiome; a score of 4 or more may be indicative of moderate dysbiosis, with a score of 7-10 being indicative of BV. Thus, "modulating" may include maintaining a Nugent score of 0-3 or reducing a Nugent score, e.g. reducing the Nugent score by a value of at least 1, 2, 3 or 4, and/or reducing the Nugent score to a score of no more than 3.

There is provided a Lactobacillus crispatus strain as provided herein and/or a composition as provided herein for use in therapy. The therapy may, e.g., be the treatment of dysbiosis, which may, e.g., be selected from dysbiosis of the gut, skin, oral cavities, urethra, vulva, vagina, uterus, and/or placenta. Dysbiosis of the vulva, vagina, uterus, and/or placenta is of particular interest. Thus, the therapy may, e.g., be the treatment and/or prophylaxis of a condition selected from vaginosis (such as bacterial vaginosis), vulval and/or vaginal yeast infection (such as candidiasis), villitis, vaginitis and/or urinary tract infection. Preferably, the therapy is the treatment and/or prophylaxis of a condition selected from bacterial vaginosis (BV) and vulvovaginal candidiasis (WC). Dysbiosis is an imbalance in the microbiota characterised in an increase in the proportion of dysbiotic bacteria, and/or a decrease in the proportion of beneficial bacteria.

Dysbiotic bacteria are those that are typically associated with dysbiosis if present in increased proportions in the microbiome. Examples of vaginal dysbiotic bacteria include Gardnerella vaginalis, Atopobium vaginae, Prevotella sp., Mobiluncus mulieris, and Candida sp.

Beneficial bacteria include probiotic bacteria.

Dysbiosis has been associated with an increased risk for infertility, spontaneous abortion, and preterm birth. Thus, the Lactobacillus crispatus strain as provided herein and/or a composition as provided herein may be used to improve the reproductive health of a subject, particularly a woman.

The terms "therapy" or "treatment" as used herein include prophylactic therapy, which may result in the prevention of disease, as well a therapeutic treatment of an established disorder. The terms "therapy" and "treatment" include combating or cure of disease but also include the controlling, reduction or alleviation of disease or one or more of the symptoms associated therewith, and/or avoiding the recurrence of the disease. In the methods of the present invention, the preferred symptom that is controlled, reduced or alleviated is discharge, discomfort and/or itching.

Also provided is a method of treating or preventing a condition selected from dysbiosis, vaginosis, vulval and/or vaginal yeast infection, villitis, vaginitis and/or urinary tract infection, said method comprising administering to a subject in need thereof an effective amount of a Lactobacillus crispatus strain provided herein and/or a composition as provided herein. Preferably, the condition is selected from bacterial vaginosis (BV) and vulvovaginal candidiasis (WC).

Also provided is a method of modulating the microbiome, e.g. vaginal microbiome of a subject, said method comprising administering to a subject in need thereof an effective amount of a Lactobacillus crispatus strain provided herein and/or a composition as provided herein.

An "effective amount" as used herein can refer to a therapeutically effective amount or a prophylactically effective amount depending on the nature of the treatment. A therapeutically effective amount can be considered to be an amount necessary (at appropriate dosages and administration regimes) to achieve the desired therapeutic result. A prophylactically effective amount can be considered to be an amount necessary (at appropriate dosages and administration regimes) to achieve the desired prophylactic result. The amounts are likely to vary depending on the weight, age and sex of the patient as well as the severity of the disease.

Suitable dosages may be readily determined, but a suitable dosage may, e.g. be $10^6$-$10^{15}$ CFU (colony forming units) per dose, e.g. $10^7$-$10^{10}$ or $10^8$-$10^9$ or $10^9$-$10^{11}$, e.g. about $10^9$ or $10^{10}$ CFU per dose. The frequency of consumption or administration of the composition, e.g. of a dose as set out above, may, e.g., be twice daily, daily, every other day, twice weekly, once a week, twice a month or once a month. Consumption/administration may be for a short period or on-going. For example, the dose may be consumed/administered daily for a period of about 1-12 weeks, e.g. 1-6, 2-4 or 1-2 weeks. A break in consumption/administration may then be taken, if appropriate, e.g. for about 1-12, 2-4 or 1-2 weeks, after which the consumption/administration may be resumed, if appropriate. For example, consumption/administration may be carried periodically, such that it is carried out for a suitable period at regular intervals, e.g. 1-3 weeks per month.

A typical daily dosage may depend on the weight of the subject. The skilled person will appreciate that suitable dosages may be achieved by administering a single dosage unit per day, or by administering two or more lower dosage units per day.

The invention will now be described in more detail by reference to the non-limiting Tables, Figures and Examples, in which

Fig. 1 is a graph showing the shelf-life of the lyophilized strains at -20°C, wherein the strains are DSM 32716, DSM 32717, DSM 32718, DSM 32719, DSM 32720 and DSM 32715;

Fig. 2 Is a Pulse-field gel electrophoresis (PFGE) profile of the *L. crispatus* strains provided herein as determined with restriction enzyme *Apal*; lines1-6: DSM 32716, DSM 32717, DSM 32718, DSM 32719, DSM 32720, and DSM 32715; M-PFGE marker (CHEF DNA Size Standard 5 kb, Bio-Rad);

Fig. 3 Is a schematic of the clinical trial scheme, wherein G =group; v = visit; w = week

Fig. 4 shows Nugent score values distribution during capsules intake for 1 week (median, IQR);

Fig. 5A-E are graphs showing correlations between the bacterial counts and Nugent score in vaginal samples, wherein

A shows the correlation between total bacterial counts and Nugent score;

B shows the correlation between *G. vaginalis* counts and Nugent score;

C shows the correlation between total lactobacillus counts and Nugent score;

D shows the correlation between *L.crispatus* counts and Nugent score; and

E shows the correlation between *A.vaginae* counts and Nugent score.

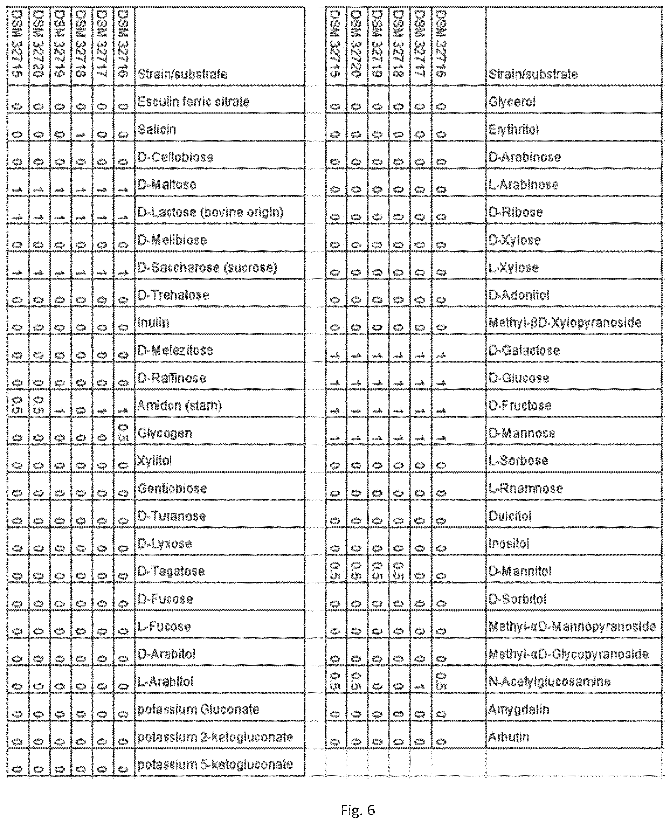

Fig. 6 is a Table of the results of the API 50 CH identification system used to analyse the *L. crispatus* strains

## EXAMPLES

### *Overview*

6 (six) novel bacterial strains of *Lactobacillus crispatus* were isolated from vaginal secretions of healthy women. The *Lactobacillus* strains were identified according to the characteristic morphology of colonies and cells and through molecular identification.

The strains were analysed for several functional properties, including lactic acid and hydrogen peroxide production, antagonistic activity against various urogenital tract pathogens, biochemical profile, proteome pattern, autoaggregation, and adhesive ability. The safety of the strains was also assessed by analyzing haemolytic activity and transferrable antibiotic resistance.

Further properties, such as growth profiles and shelf life after lyophilisation, were also tested.

Clinical trials were also carried out, showing an advantageous effect.

### Example 1A Detection of hydrogen peroxide

The lactobacilli strains were tested for the production of $H_2O_2$ on a tetramethylbenzidine (TMB, Sigma) agar plate (method according to Eschenbach et al. Prevalence of hydrogen peroxide-producing Lactobacillus species in normal women and women with bacterial vaginosis. J Clin Microbiol 1989; 27 (2):251-256). The lactobacilli strains were plated on TMB agar consisting of tetramethyl-benzidine, horseradish peroxidase, brucella agar base, hemin, starch and vitamin K, and incubated for 48 h at 37°C in anaerobic conditions. After exposure to ambient air, hydrogen peroxide producing colonies turned blue. Change of color was assessed semi-quantitatively after 30 min. Color intensity was designated as follows: - (negative), + (weakly positive), ++ (moderately positive), and +++ (strongly positive). The results are presented in **Table 1.**

### Example 1B Detection of lactic acid

The production of lactic was estimated by gas chromatography as described in Holdeman et al. (Anaerobe Laboratory Manual. Blacksburg, VA: Virginia Polytechnic Institute and State Laboratory, 1977.) Analysis was performed after cultivation of lactobacilli at 37°C in modified MRS broth for 48 h in an anaerobic glove chamber (Concept 400, Biotrace, Bridgend, UK) with a gas mixture of $CO_2/H_2/N_2$:5/5/90%. The samples were then centrifuged at 6000 g for 10 min. Lactic acid methylate samples were prepared by adding 0.4 g sodium chloride, 0.2 ml 50% sulfuric acid and 2 ml methanol in 1 ml prepared culture broth. Tubes were heated at 60°C for 1.5 h, added 1 ml water and 0.5 ml chloroform.

The emulsion form was centrifuged 3000 rpm for 3 min. One microliter of the chloroform sample was injected into the GC column. The HP Chemical Station for GC system (A.06 revision) was used. The gas chromatograph (Hewlett-Packard 6890, USA) was equipped with a hydrogen flame ionization detector, an autosampler (model 7683) and a capillary GC column HP-INNOWax (Hewlett-Packard, USA) was 15 m X 0.25 mm (i.d.), coated with cross-linked polyethylene glycol (film thickness 0.15 mm). The detector temperature was 250°C and the injector temperature was 200°C, respectively. The oven temperature program was a gradient system; the initial temperature was 60°C for 1 min that was increased to 120°C at a rate of 10°C/min and held there for 10 min. Lactic acid (Aldrich, USA) was used to prepare standards. Methylated stock solutions were a different concentration of each acid, namely 0.15, 0.3, 0.6, 1.2, 2.4, 3.6, 4.8 mg/ml. Each standard solution was injected in duplicate to obtain its retention time and area under the curve. The chromatographic peak areas were integrated with a Hewlett-Packard networking integrator. The results are presented in **Table 1**.

**Table 1** Production of lactic acid and $H_2O_2$ by *L. crispatus* strains.

| Strain | Amount of produced lactic acid (mg/ml) | Production of $H_2O_2$ |
|---|---|---|
| DSM32716 | $17.79 \pm 0.20$ | + |
| DSM32717 | $19.02 \pm 0.01$ | ++ |
| DSM32718 | $15.2 \pm 0.11$ | + |
| DSM32719 | $18.59 \pm 0.08$ | + |
| DSM32720 | $18.64 \pm 0.18$ | +++ |
| DSM32715 | $15.71 \pm 0.05$ | +++ |
| The production of $H_2O_2$ was assessed semi-qualitative and evaluated as follows: - (negative), + (weakly positive), ++ (moderately positive), and +++ (strongly positive). | | |

## Example 2 Analysis of carbohydrate utilisation

The API 50 CH identification system (bioMérieux, Inc., Marcy l'Etoile, France) was used according to manufacturer's instructions. The results are presented in **Fig. 6.**

## Example 3 Detection of haemolytic activity

A single line of lactobacilli culture (grown in MRS broth for 48 h) was streaked onto blood agar plates containing either human, sheep or horse blood. The haemolytic activity of the strain was verified visually after 24 h and 48 h of incubation in anaerobic (90% $N_2$, 5% $CO_2$, 5% $H_2$) environments. The blood agar plates were examined for signs of β-haemolysis (clear zones around colonies), α-haemolysis (green halo around colonies) or γ-haemolysis (no zones around colonies) (Pfaller et al. Algorithm for Identification of Aerobic Gram-Positive Cocci, In Manual of Clinical Microbiology. American Society for Microbiology; 2007). A *Staphylococcus aureus* strain (ATCC 25923) and a *Streptococcus pyogenes* strain (ATCC 19615) were used as positive controls.
No haemolysis was seen for any of the six L. crispatus strains.

## Example 4

## A) Testing of autoaggregation

Autoaggregation was tested according to a modified method previously described in Kos et al. Adhesion and aggregation ability of probiotic strain Lactobacillus acidophilus M92. J Appl Microbiol 2003; 94 (6):981-987. The strains were incubated in MRS broth (de Man Rogosa Sharp, Oxoid Ltd., Basingstoke, UK) for 18 hours at 37°C in anaerobic conditions ($CO_2/H_2/N_2$: 5/5/90%). Thereafter, the strains were washed twice in PBS buffer and resuspended in PBS to the concentration of $10^8$ CFU/ml (McFarland 3.0). The suspension was vortexed for 10 seconds. Next, 0.2 ml of the upper layer was pipetted into the wells of the microtiter plate and optical density (OD) at 600 nm was measured ($A_0$). The second OD detection ($A_t$) was performed after the suspension was stored at room temperature for 2 hours. The autoaggregation was detected with the following calculation:

$$\text{Autoaggregation (\%)} = [1-(A_t/A_0)] \times 100.$$

Any autoaggregation was also estimated visually. The autoaggregation was scored positive when clearly visible sand-like particles were formed by the aggregated cells, and settled in the bottom of the tubes, leaving a clear supernatant. The results were scored on a 3 point scale depending on the time of the autoaggregation. If the autoaggregation was detected within 15 min, the reaction was scored 3; within 15-30 min the score was 2, and within 30-60 min, the reaction was scored with 1 (Bujnakova and Kmet, Aggregation of animal lactobacilli with 0157 enterohemorrhagic Escherichia coli. J Vet Med B Infect Dis Vet Public Health 2002; 49 (3):152-154). All measurements were performed three times and the average of separate detections was calculated.

The results are presented in **Table 2**.

**B) Testing of adhesive properties**

The *L. crispatus* strains were tested for their ability to adhere to HeLa cells. *Lactobacillus rhamnosus* GG (ATCC 53103) was used as a positive control. For adhesion testing 2 ml of HeLa cell suspension was added to each well in a 6-well tissue culture plate at a concentration of $2 \times 10^5$ cells/ml and incubated in a 5% $CO_2$ atmosphere at 37°C for 48 hours until the cells were grown to approximately 60% confluence (Kaewnopparat et al. In vitro probiotic properties of Lactobacillus fermentum SK5 isolated from vagina of a healthy woman. Anaerobe 2013; 22:6-13). After 48-hours growing, cells were washed twice with PBS. Lactobacilli were incubated for 48 hours at 37°C in liquid MRS (0.1 ml of seedbank in 4 ml of MRS), centrifuged at 3000 rpm for 4 minutes and washed twice with PBS. Lactobacilli suspension was prepared in PBS according to McFarland 3.0. 0.1 ml of lactobacilli suspension was added to 0.9 ml DMEM/Ham's F-12 with stable glutamine (GE Healthcare), and incubated in 1 ml well at 37°C for 1 hour in 10% $CO_2$. Next, cells were washed in 1 ml of PBS five times and 1 ml of 0.05% Triton X100 for 10 min was added. Applying ten-fold dilution method, lactobacilli were seeded onto MRS agar, after incubation the colonies were counted and expressed in % of adhesion.

$$\text{Adhesion (\%)} = (N_1/N_0) \times 100\%, \text{ where } N_1 - \text{the amount of adhered bacteria } (\log_{10}\text{CFU/ml});$$
$$N_0 - \text{the amount of applied bacteria } (\log_{10}\text{CFU/ml}).$$

All lactobacilli strains were tested in 2 repeats for 3 times. The results are presented in **Table 2.**

**Table 2**. Autoaggregation and adhesion of novel *L. crispatus* strains

| Strain | Autoaggregation (range, median) | Visual auto-aggregation (%) | Adhesion (%) (mean, sd) |
|---|---|---|---|
| DSM32716 | 0 - 7.5/4.0 | 0 | 91.9 ± 3.7 |
| DSM32717 | 0 - 10.2/9.6 | 0 | 92.4 ± 2.3 |
| DSM32718 | 2.9 - 66.9 / 36.9 | 0-2/ 1 | 92.8 ± 2.7 |
| DSM32719 | 0 - 28.8 / 0.0 | 0 | 95.4 ± 2.1 |
| DSM32720 | 3.6 - 14.5 / 11.5 | 0 | 93.3 ± 3.3 |
| DSM32715 | 6.3 - 8.4 / 7.1 | 0 | 90.4 ± 1.9 |

**Example 5 Antibiotic susceptibility testing**

The susceptibility to ampicillin, chloramphenicol, clindamycin, erythromycin, gentamycin, kanamycin, metronidazole, nitrofurantoin, norfloxacin, streptomycin, tetracycline, trimethoprim/sulfamethoxazole and vancomycin was assessed according to European Union Commission recommendations for probiotic safety. The minimum inhibitory concentrations (MICs) were determined by the Etest® method (BioMerieux, Marcy l'Etoile, France). The assay was performed by suspending individual colonies (picked up from fresh cultures on LSM agar [90% isosensitest (Oxoid Ltd., Basingstoke, UK) and 10% MRS (Oxoid)] plates incubated for 24 h at 37°C in an anaerobic glove chamber (Concept 400, Biotrace, Bridgend, UK) to McFarland standard 1 in sterile 0.85% NaCl solution. The suspension was swabbed in three directions on LSM agar and allow to dry before applying the Etest strips. Plates were incubated at 37°C for 48 h under anaerobic conditions before final results were recorded. MICs were read directly from the test strip according to the instructions of the manufacturer. Strains showing MICs less than EFSA's breakpoints were considered sensitive; otherwise, they were resistant. The results are presented in **Table 3.**

**Table 3** Antibiotic susceptibility of the novel *L. crispatus* strains.

| Strain | MIC values ($\mu$g/ml) | | | | | | | | | | | | |
|---|---|---|---|---|---|---|---|---|---|---|---|---|---|
| | AMP | CHL | CLI | ERY | GEN | KAN | MZH | NIT | NOR | STR | TET | SXT | VAN |
| DSM32716 | 0.5 | 0.75 | 0.016 | 0.016 | 2 | **>256** | **>256** | 0.5 | **>256** | 6 | 0.25 | **>32** | 0.25 |
| DSM32717 | 0.38 | 1.5 | 0.016 | 0.094 | 6 | **>256** | **>256** | 1.0 | **>256** | **16** | 0.75 | **>32** | 0.5 |
| DSM32718 | 0.38 | 0.5 | 0.032 | 0.016 | 1.5 | **48** | **>256** | 0.75 | **>256** | 4 | 0.25 | **>32** | 0.25 |
| DSM32719 | 0.5 | 1.5 | 0.016 | 0.047 | 2 | **128** | **>256** | 1.0 | **>256** | **24** | 0.5 | **>32** | 0.5 |
| DSM32720 | 0.25 | 0.75 | 0.016 | 0.023 | 2 | **96** | **>256** | 0.5 | **48** | 4 | 0.25 | **>32** | 0.25 |
| DSM32715 | 0.25 | 1.5 | 0.047 | 0.023 | 2 | **>256** | **>256** | 1.5 | **>256** | 8 | 0.38 | **>32** | 0.38 |
| MIC values of the strains measured by E-test, bold indicates resistant strain. AMP-ampicillin; CHL-chloramphenicol; CLI-clindamycin; ERY-erythromycin; GEN-gentamicin; KAN-kanamycin; MZH-metronidazole; NIT-nitrofurantoin; NOR- norfloxacin; STR-streptomycin; TET- tetracycline; SXT- trimethoprim-sulfamethoxazole; VAN-vancomycin | | | | | | | | | | | | | |

## Example 6 Detection of antimicrobial resistance genes

Total DNA of *Lactobacillus* strains was extracted using a QIAamp DNA Mini Kit (Qiagen GmbH, Hilden, Germany) following the manufacturer's protocol. The PCR assay mix (total volume, 50 $\mu$l) contained 20 pmol of each primer, 1XPCR buffer (Fermentas, Lithuania), each deoxynucleoside triphosphate at a concentration of 200 $\mu$M, and 2U Taq DNA polymerase. A 50 ng portion of purified total DNA was used as a template. In a first PCR assay, *tet* genes encoding ribosomal protection proteins (RPP) were detected with degenerated primers DI and DII (Clermont et al. New tetracycline resistance determinants coding for ribosomal protection in streptococci and nucleotide sequence of tet(T) isolated from Streptococcus pyogenes A498. Antimicrob Agents Chemother 1997; 41 (1):112-116).

If positive, additional PCR assays were performed with primers specific for the *tet*M, *tet*O, *tet*S, *tet*W and *tet*Q genes. All *Lactobacillus* strains were tested for the presence of the tetracycline efflux genes *tet*K and tetL. Polymerase chain reaction amplicons for tet genes were performed in an Eppendorf PCR System (Eppendorf AG, Hamburg, Germany) with the following programs: initial denaturation at 94°C for 5 min; 35 cycles of 94°C for 1 min, corresponding annealing temperature for 1 min, and 72°C for 2 min; and a final extension step at 72°C for 10 min. Amplification specifications for integrase Int1 were as follows: 94°C for 5 min, followed by 25 cycles of 94°C for 30 s, 55°C for 30 s, and 72°C for 30 s, and a final extension at 72°C for 7 min. Amplicons were electrophoresed on 1.5% agarose gel, and a 1-kb or 100 bp ladders (Fermentas) were used as a molecular size marker. The results are presented in **Table 4.**

**Table 4** Detected antimicrobial resistance genes of the novel *L. crispatus* strains.

| Strain | *erm*(B) | *tet*(M) | *tet*(S) | *tet*(O) | *tet*(K) | *tet*(L) | RPP |
|---|---|---|---|---|---|---|---|
| DSM32716 | - | - | - | - | - | - | - |
| DSM32717 | - | - | - | - | - | - | - |
| DSM32718 | - | - | - | - | - | - | - |
| DSM32719 | - | - | - | - | + | - | + |
| DSM32720 | - | - | - | - | - | - | - |
| DSM32715 | + | - | - | - | - | - | - |
| +, presence, -, absence; RPP, *tet* genes encoding ribosomal protection proteins | | | | | | | |

## Example 7 Detection of antagonistic activity

### A) Testing of antagonistic activity against urinary E.coli pathogens

For detection of antimicrobial activity of isolated lactobacilli strains against urinary tract pathogens the following bacteria were used: *E. coli* ATCC 700414, *E. coli* ATCC 700336 and three *E. coli* strains that were previously isolated from women with bacterial vaginosis.

The inhibitory effect of vaginal strains on *E. coli* strains was determined by a modified agar spot method (Jacobsen et al. Screening of probiotic activities of forty-seven strains of Lactobacillus spp. by in vitro techniques and evaluation of the colonization ability of five selected strains in humans. Appl Environ Microbiol 1999; 65 (11):4949-4956). Briefly, the tested lactobacilli cultures were spotted (1 $\mu$l) on the surface of MRS agar (20 ml) and incubated anaerobically for 24 h at 37°C. An overnight culture of *E. coli* (approximately 8 x 10^8 cells) was mixed with soft agar (0.7%) nutrient agar (20 ml) (Oxoid Ltd., Basingstoke, UK), and poured over the plate in which lactobacilli were grown. After anaerobic incubation for 24 h at 37°C, the plates were checked for inhibition zones around *Lactobacillus* spots and their diameters were recorded. All tests concerning the detection of antimicrobial activity were repeated three times in three independent experiments. The results are presented in **Table 5.**

**Table 5** Antimicrobial activity of novel strains against different uropathogenic *E. coli* (inhibition zones in mm, expressed as mean, sd)

| Strain | *E. coli* RTN038 | *E. coli* RTN047 | *E. coli* RTN100 | *E. coli* ATCC 700414 | *E. coli* ATCC 700336 |
|---|---|---|---|---|---|
| DSM32716 | 9.5 ± 0.11 | 9.8 ± 0.59 | 7.9 ± 0.59 | 10.3 ± 0.11 | 11.0 ± 0.35 |
| DSM32717 | 8.3 ± 0.59 | 10.1 ± 1.00 | 10.1 ± 0.53 | 9.1 ± 0.71 | 10.0 ± 0.65 |
| DSM32718 | 8.7 ± 0.23 | 10.6 ± 0.42 | 9.2 ± 0.12 | 9.8 ± 0.00 | 9.5 ± 0.00 |
| DSM32719 | 9.3 ± 0.12 | 12.0 ± 0.24 | 9.4 ± 0.01 | 10.3 ± 0.24 | 10.5 ± 0.18 |
| DSM32720 | 8.5 ± 0.06 | 11.3 ± 0.00 | 11.8 ± 0.59 | 10.0 ± 0.01 | 11.3 ± 0.01 |
| DSM32715 | 9.3 ± 0.00 | 12.1 ± 0.35 | 12.2 ± 0.18 | 11.0 ± 0.53 | 11.7 ± 0.12 |

**B) Testing of antagonistic activity against Candida spp**

For detection of anti-candidal activity of isolated lactobacilli strains the following strains were used: *Candida albicans* ATCC 32032, *Candida albicans* RTN 071 and *Candida glabrata* RTN 009. Anti-candidal activity was detected by a modified agar overlay technique (Strus et al. The in vitro activity of vaginal Lactobacillus with probiotic properties against Candida. Infect Dis Obstet Gynecol 2005; 13 (2):69-75). Briefly, *Lactobacillus* isolate was cultured in MRS broth for 24 h at 37°C in anaerobic conditions. Afterwards, lactobacilli were cultured in the middle of the MRS agar plate as a stripe 2 cm wide using a sterile cotton swab. The plates were incubated in anaerobic conditions at 37°C for 48 h. The *Lactobacillus* spp growth was then overlaid with melted MRS agar. After solidification of the agar overlay, a suspension of *C. albicans* or *C. glabrata* (obtained overnight culture of in Sabouraud agar (Oxoid)), in sterile saline with turbidity 0.5 MacFarland's score was streaked over the agar surface with a cotton swab. The plates were preincubated at 4°C for 4 h in a refrigerator, then kept at 37°C for 24 h in aerobic conditions and left for 24 h at room temperature before reading the results. After adequate growth, the zones of inhibition of *Candida* spp were evaluated using a measure of *Lactobacillus* spp streak line with inhibition zone and divided by 2. The results are presented in **Table 6**.

**Table 6** Anti-candidal activity of novel strains

| Strain | *C. albicans* ATCC 32032 | *C. albicans* RTN071 | *C. glabrata* RTN 009 |
|---|---|---|---|
| DSM32716 | intermediate | high | high |
| DSM32717 | high | high | low |
| DSM32718 | high | high | intermediate |
| DSM32719 | high | high | intermediate |
| DSM32720 | high | high | high |
| DSM32715 | low | high | high |
| Anti-candidal activity was detected by a modified agar overlay technique. The inhibitory effect of lactobacilli was ranked as high (>3.5 mm), intermediate (2.0-3.5 mm) and low (<2.0 mm) | | | |

**C) Testing of antagonistic activity against Gardnerella vaginalis**

For detection of anti-*Gardnerella vaginalis* activity of the novel strains, cell-free supernatants of the novel strains and a reference strain *Gardnerella vaginalis* ATCC 14018 (DSM 4944) and one clinical isolate of *G. vaginalis* as target bacteria were used. Culture supernatants of the novel strains were prepared by cultivation in MRS broth anaerobically at 37°C for 48 h. Thereafter, removal of the cells by centrifugation at 6000 g for 15 minutes was done. The pH of each supernatant was measured (Hanna Instruments HI 9024C, Singapore) and supernatants were sterilized using 0.20 μm millipore filters (Sarstedt, Nümbrecht, Germany). Natural cell-free culture supernatants of the novel strains were examined for their antibacterial activity on 96-well microtiter plates. *G. vaginalis* strains were grown on BD Gardnerella Selective Agar with 5% human blood in anaerobic environment for 48 h. G. *vaginalis* colonies were suspended in sterile saline to achieve a turbidity of about 0.5 McFarland.

Further, *G. vaginalis* suspensions (20 μl), 5% human serum (Sigma-Aldrich Co, USA) in Schaedler broth (Oxoid) (150 μl) and natural cell-free supernatant (30 μl) were transferred to a 96-well microplate (served as test well). *G. vaginalis* suspensions (20 μl) and 5% human serum in Schaedler broth (180 μl) transferred into microplate served as the positive control (control well). Growth and growth inhibition of *G. vaginalis* was determined by monitoring the changes in optical density (OD) at 600 nm as measured with a absorbance microplate reader (Sunrise, Tecan, Austria) after 0 and 48 h incubation. Readings from the 0 h incubation time were used to determine the absorbance due to media and inoculum.

The percentage of reduction in growth of target bacteria due to cell-free supernatant was calculated as per the following formula:

% of reduction in growth = (OD value of control well – test well / OD value of control well) x 100.

Experiments were repeated three times with two replicates. The results are presented in **Table 7.** The inhibition of *G. vaginalis* growth in percent was ranked as high (>78.0%), intermediate (67.3-77.0%) or low (<67.2%).

**Table 7** Antimicrobial activity of novel strains against *G. vaginalis* strains.

| Strain | *G. vaginalis* (clinical strain) | *G. vaginalis* ATCC 14018 |
|---|---|---|
| DSM32716 | low | low |
| DSM32717 | high | high |
| DSM32718 | intermediate | intermediate |
| DSM32719 | intermediate | intermediate |
| DSM32720 | intermediate | intermediate |
| DSM32715 | intermediate | intermediate |

**Example 8 Analysis of growth kinetics**

The novel strains were incubated in aerobic conditions at 37°C for 24 hours in 40 ml of liquid MRS on shaker with speed of 150 rpm. After incubation, 10% of glycerol was added to the aliquots of 0.5 ml, and stored at -80°C. The density of the suspension was detected with bacteriologic seeding dilution method. Next, growth curves were measured for all strains. The volume of the suspension in liquid MRS was calculated according to density of $10^5$ cfu/ml, and suspended into each well of the 96 well plate. These well plates were incubated in aerobic and anaerobic conditions at 37°C. During aerobic cultivation the time points for measuring growth curves were 0, 4, 18, 20, 22, 24, 28 and 48 hours, and in case of anaerobic conditions at 0, 4, 24, 28 and 48 hours. The density of bacterial growth was detected with bacteriologic seeding dilution method, and the spectrophotometrically at 600 nm. At the same time all the strains were incubated in microplate reader with integrated incubator (Infinite 200Pro, Tecan,) in aerobic conditions at 37°C for 29 hours. Optical density (OD) at 600 nm was measured with the interval of 30 minutes with the preceding shaking of 10 seconds with the amplitude of 5mm. All lactobacilli strains were tested in 4 repeats for 2 times. Good growth kinetics were observed.

**Example 9** *Analysis of shelf-life*

Lyophilized powders of the novel *L. crispatus* strains were made and the shelf-life thereof was tested at -20°C. To check the viability, 0.1 g of powder was taken, suspended in PBS (1:9) and line of dilutions was prepared ($10^{-1}$ - $10^{-10}$). The dilutions were cultured on MRS agar plates and incubated in anaerobic conditions for 48 h. The colonies were counted in each plate and the density of the powder (number of bacteria per g or per ml) was calculated according to the formula (ISO7218):

$$N = \frac{\Sigma C}{v(n_1 + 0,1\, n_2)d}$$

where:

$\Sigma C$    is the sum of the colonies counted on all the plates;
V    is the volume of inoculum applied to each dish, in ml;
$n_1$    is the number of plates counted at the first dilution;
$n_2$    is the number of plates counted at the second dilution;
d    is the dilution from which the first counts were obtained.

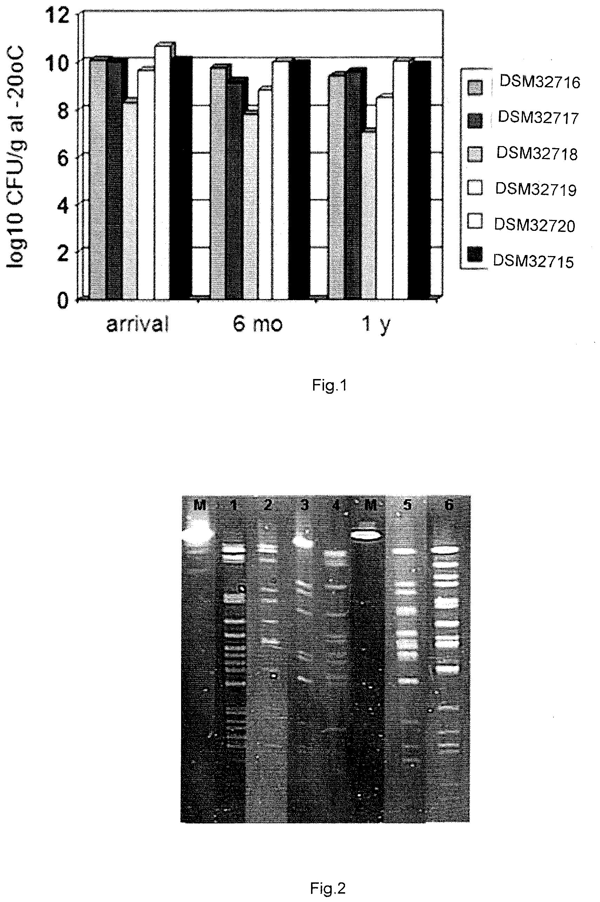

The results are given on **Figure 1.** Viability remained unchanged at -20°C within one year.

**Example 10 Analysis of fingerprint profile by pulse-field gel electrophoresis (PFGE)**

The novel strains were grown to an $A_{620}$ of 0.530 in MRS broth. Cells were harvested from 1.5 ml of culture, washed with 75mM NaCl, 25mM EDTA (pH 7.4), and suspended in 150 $\mu$l of the same buffer. The suspension was heated in 50°C, and 150 $\mu$l of 2% agarose in 0.5XTBE buffer at the same temperature was added before solidifying the suspension. The agarose plugs were incubated overnight at 37 °C in lysis buffer, 50mM EDTA (pH 8.5), 0.5% Na-laurylsarcosine, 0.2% Na-deoxylate, containing 2mg of lysozyme (Sigma) per ml and 10U of mutanolysin (Sigma) per ml and them incubated overnight at 53 °C in solution containing 10 mM Tris, 0.5M EDTA (pH 8.5), 1% sodium dodecyl sulfate and 2 mg of proteinase K per ml. The agarose blocks were washed three times with 75mM NaCl, 25mM EDTA (pH 7.4). Restriction enzyme digestion with *ApaI* was performed overnight at 37 °C. Electrophoresis was carried out with a CHEF DR II (Bio-Rad, Hercules, Calif) with 0.5XTBE buffer. The pulse time was 1 to 15 s, the current was 6V/cm, the temperature was 14°C, and the running time was 18 hours. The agarose gel was stained with etidium bromide (0.5$\mu$g/ml) and visualized under UV light (Simpson et al. 2003. Genomic diversity and relatedness of bifidobacteria isolated from a porcine cecum. J Bacteriol, 185, 2571-81). The results are given in **Figure 2.** All the strains were different from each other.

**Example 11 Detection of proteome**

Proteomes of strains DSM 32716, DSM 32717, DSM 32718 and DSM 32720 were mass-spectrometrically determined and analyzed by bioinformatic means, focusing on the proteins with probiotic potential.
A "bottom-up" protein determination method was used, for which the investigated proteins were proteolytically fragmented into peptides (using trypsin) prior to determination of mass-spectra. Mass-spectrometrically produced raw data of proteolytic peptides were interpreted using MaxQuant software, with which the instrumentally identified mass spectra or tandem mass spectra (MS/MS) were matched with known proteins expressed in organism of interest (*Lactobacillus crispatus* reference strain ST1) using specific algorithms (Schaab et al. 2012, Analysis of High Accuracy, Quantitative Proteomics Data in the MaxQB Database. Mol Cell Proteomics, M111.014068; Koenig et al. 2008, Robust prediction of the MASCOT score for an improved quality assessment in mass spectrometric proteomics. J. Proteome Res. 7(9): 3708-17). For bioinformatic analysis of the received proteomic data, central protein database Uniprot was used. In order to gather proteins with probiotic importance from the whole proteomes, functional annotations based on Gene Ontologies were used. Proteins of interest were aligned against reference proteome ST1 using BlastP application in order to obtain suitable nomenclature of the data. To gather into collection additional proteins with active probiotic importance, protein sequences of bacteriocins belonging to other *Lactobacillus* species (*L. helveticus, L. paracasei, L. kunkeei, L. sakei, L. divergens, L. johnsonii, L. gasseri, L. plantarum, L. acidophilus, L. rhamnosus*) were searched from databases UniProt and BactiBase. The found bacteriocin sequences were aligned using BlastP application against reference proteome *L. crispatus* ST1.
The individual strains harboured 1006 to 1043 different proteins and peptides. Three strains expressed strain-specific exclusive proteins (DSM 32716, n=1; DSM 32717, n=1; DSM 32720, n=38). In all strains, proteins associated with probiotic activity were expressed, allowing us to use them for balancing urogenital microbiota (**Table 8**).
The presence of strains with distinct expression patterns of probiotically important proteins gives us the opportunity to create combined probiotic formulas in which strains with different properties are assembled, in order to achieve maximum effectiveness towards patients with different clinical symptoms, geno- and phenotypical individuality and variable vaginal microbiota composition.

**Table 8** Proteins with probiotic potential in new Lactobacillus crispatus strains

| | Protein | Gene | Peptide count | | | |
|---|---|---|---|---|---|---|
| | | | DSM 32716 | DSM 32717 | DSM 32718 | DSM 32720 |
| **Bacteriocins,bacteriocin transporters and proteins similar to bacteriocins** | | | | | | |
| Class III | Helveticin-J | LCRIS_ 01534 | 3 | 1 | 2 | 7 |
| | Helveticin-J | LCRIS_ 00011 | 1 | 1 | 2 | 7 |
| | Helveticin biosynthesis cyclodehydratase | LCRIS_ 01052 | 9 | 10 | 10 | 10 |
| | Helveticin secretion accessory protein | LCRIS_ 00701 | 0 | 0 | 0 | 1 |
| | Helveticin biosynthesis cyclodehydratase | LCRIS_ 01305 | 3 | 12 | 11 | 1 |
| Class IIb | Lactococcin | LCRIS_ 01239 | 0 | 0 | 0 | 6 |
| | Lactacin | LCRIS_ 00808 | 2 | 4 | 1 | 9 |
| | Gassericin | LCRIS_ 01823 | 1 | 1 | 1 | 17 |
| | Gassericin/Plantaricin | LCRIS_ 01824 | 1 | 1 | 2 | 9 |
| | Glycocin | LCRIS_ 00038 | 3 | 3 | 5 | 1 |
| | Glycocin/Plantaricin | LCRIS_ 00109 | 0 | 0 | 0 | 3 |
| | Plantaricin | LCRIS_ 00194 | 10 | 11 | 10 | 18 |
| Class I/IIc/IId | Nisin (class I) | LCRIS_ 00099 | 0 | 1 | 0 | 0 |
| | Lacticin (class I) | LCRIS_ 01673 | 17 | 17 | 22 | 22 |
| | Acidocin (class IIc) | LCRIS_ 00919 | 4 | 3 | 3 | 2 |
| | Rhamnosin (class IId) | LCRIS_ 00224 | 14 | 14 | 14 | 4 |

(continued)

| | Protein | Gene | Peptide count | | | |
|---|---|---|---|---|---|---|
| | | | DSM 32716 | DSM 32717 | DSM 32718 | DSM 32720 |
| **Bacteriocins,bacteriocin transporters and proteins similar to bacteriocins** | | | | | | |
| Bacteriocin transporters | Bacteriocin transporter | LCRIS_01639 | 0 | 1 | 0 | 0 |
| | Bacteriocin transporter | LCRIS_01845 | 4 | 3 | 2 | 3 |
| | ABC-type antimicrobial peptide transporter | LCRIS_01868 | 10 | 7 | 9 | 3 |
| | ABC-type antimicrobial peptide transporter | LCRIS_01306 | 9 | 11 | 11 | 18 |
| **Other proteins with active probiotic effect** | | | | | | |
| Lysins | Hemolysin | hlyIII | 1 | 1 | 1 | 1 |
| | Lysin | LCRIS_01140 | 14 | 12 | 13 | 2 |
| | Lysin | LCRIS_01354 | 12 | 12 | 12 | 0 |
| | Enterolysin | LCRIS_01195 | 2 | 3 | 3 | 4 |
| | Enterolysin | LCRIS_01224 | 6 | 6 | 6 | 5 |
| | Hemolysin | tlyA | 10 | 11 | 11 | 0 |
| | Lysin | LCRIS_01961 | 21 | 22 | 22 | 4 |
| Other proteins | CRISPR endonuclease | LCRIS_01692 | 0 | 0 | 0 | 5 |
| | CRISPR endonuclease | LCRIS_00040 | 3 | 4 | 4 | 1 |
| | Holin | LCRIS_00752 | 4 | 5 | 5 | 4 |
| | Holin | LCRIS_01948 | 10 | 11 | 10 | 12 |
| | Holin | LCRIS_00956 | 12 | 16 | 11 | 14 |
| | Antibiotic biosynthesis monooxygenase | LCRIS_00495 | 13 | 11 | 12 | 15 |
| | Antibiotic biosynthesis monooxygenase | LCRIS_01241 | 4 | 5 | 2 | 6 |

(continued)

| Proteins with passive probiotic effect | | | | | | |
|---|---|---|---|---|---|---|
| Adhesion and biofilm formation | Surface adhesin | LCRIS_ 01803 | 16 | 16 | 13 | 2 |
| | Aggregation factor | LCRIS_ 00491 | 11 | 11 | 11 | 11 |
| | Aggregation factor | LCRIS_ 01064 | 2 | 2 | 2 | 2 |
| | Aggregation factor | LCRIS_ 01883 | 5 | 5 | 5 | 5 |
| | Mucus-binding protein | LCRIS_ 00919 | 4 | 3 | 3 | 2 |
| | Mucus-binding protein | LCRIS_ 00029 | 2 | 7 | 0 | 0 |
| | Fibronectin-binding protein | LCRIS_ 00195 | 22 | 22 | 21 | 13 |
| | Fibronectin-binding protein | LCRIS_ 01031 | 0 | 1 | 0 | 0 |
| Response to environment al conditions | UvrABC system protein B | uvrB | 13 | 17 | 17 | 20 |
| | LexA repressor | lexA | 4 | 4 | 5 | 3 |
| | UvrABC system protein C | uvrC | 4 | 7 | 8 | 9 |
| | Protein RecA | recA | 17 | 15 | 18 | 19 |
| | UvrABC system protein A | uvrA | 25 | 31 | 36 | 33 |
| | Holliday junction ATP-dependent DNA helicase RuvA | ruvA | 9 | 9 | 9 | 9 |
| | Holliday junction ATP-dependent DNA helicase RuvB | ruvB | 3 | 4 | 5 | 4 |
| | ATP-dependent DNA helicase RecQ | recQ | 1 | 1 | 1 | 4 |
| | Thioredoxin reductase (removal of superoxide radicals) | trxB2 | 21 | 20 | 21 | 23 |
| | Peptide methionine sulfoxide reductase MsrA (response to oxidative stress) | msrA | 5 | 4 | 5 | 0 |
| | Peptide methionine sulfoxide reductase MsrB (response to oxidative stress) | msrB | 2 | 3 | 0 | 12 |
| | Chaperone protein DnaJ | dnaJ | 12 | 18 | 8 | 7 |
| Immunity | CRISPR-associated protein | LCRIS_ 01209 | 0 | 0 | 0 | 4 |
| | CRISPR-associated protein | casE | 0 | 0 | 0 | 36 |
| | CRISPR-associated protein | LCRIS_ 01213 | 0 | 0 | 0 | 13 |
| | CRISPR-associated helicase | cas3 | 2 | 3 | 1 | 0 |
| | Abortive phage resistance protein | LCRIS_ 01105 | 2 | 3 | 4 | 13 |

**Example 12 Clinical trial**

*Study information*

Forty women of reproductive age (mean age 28.9±8.8, range 20-50 years) were recruited into the trial. Absence of chronic diseases was confirmed by questionnaire and blood analysis at screening visit. In a randomized double-blind placebo-controlled crossover study the eligible participants were randomly allocated to one of four groups and had to take either (i) the study product, a capsule containing three different *L. crispatus* strains ($10^9$ CFU per strain); or (ii) placebo (sucrose) for 1 week. Two different probiotic mixtures were tested: Probiotic Mixture I (PM I, containing strains DSM 32716, DSM 32719, and DSM 32715) and Probiotic Mixture II (PM II, containing strains DSM 32717, DSM 32718, DSM 32720).

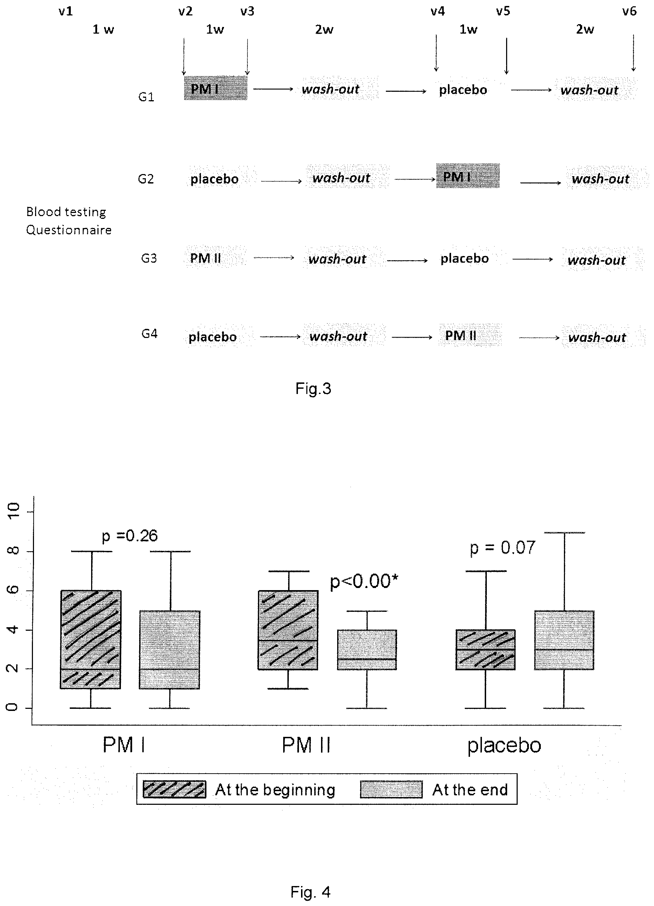

The trial scheme: the treatment period was followed by a 2-week washout period and then by a second treatment and washout period (see **Figure 3**). Individuals who first received the study product subsequently received the placebo, and *vice versa.* On each scheduled visit, the respondents were given oral and written instructions by the field coordinator before the next visit. Vaginal and stool samples were self-collected in the morning of the scheduled visit (or stool samples the night before with instructions to keep the samples inside the freezer, at -20 °C). All samples were delivered to the laboratory immediately, where they were subsequently frozen at -80 °C.

*Vaginal, gastrointestinal and general health monitoring*

Assessment of vaginal and gastrointestinal health using self-reported questionnaires was performed from the first treatment period to the second wash-out period. General gastrointestinal questions including the presence of severity of stomach pain, bloating, gastric reflux, nausea, vomiting, flatulence and stool consistency was rated using scale from no complaints to presence of severe symptoms. Vaginal discharge questions were based on markers about discharge amount, consistency, color, odor and itchiness.

The study product (probiotics capsules) appeared to be generally well tolerated. The rate of occurrence of adverse events was unrelated to assignment to either of the treatment groups (probiotic or placebo), as the number of adverse events in each group was similar. No serious adverse events were observed in either both of probiotics or placebo group (**Table 9**).

**Table 9.** Summary of gastrointestinal adverse events - end of treatment phase

| Adverse event | PM I (n=15) | | PM II (n=14) | | Placebo (n=28)[1] | |
|---|---|---|---|---|---|---|
| | Mild | Moderate | Mild | Moderate | Mild | Moderate |
| Stomach pain | 1 | 0 | 0 | 0 | 0 | 1 |
| Upper-abdominal discomfort | 1 | 0 | 0 | 0 | 0 | 1 |
| Nausea/vomiting | 0 | 0 | 0 | 0 | 1 | 1 |
| Flatulence | 0 | 2 | 0 | 0 | 3 | 0 |
| Burping | 0 | 0 | 0 | 0 | 2 | 0 |
| Defecation/bowel movement problems | 0 | 0 | 0 | 0 | 2 | 0 |
| **Total** | **2** | **2** | **0** | **0** | **8** | **3** |

[1] data of one subject was excluded, due to elevated CRP-hs during the placebo intake

There were no adverse events concerning the vaginal discharge characteristics. In self-assessment questionnaires, most of participants reported no significant changes during the 7-day period of capsule consumption. Three subjects, who consumed PM I capsules noted subjective improvement in the discharge consistency.

Hematological and inflammatory indices, metabolic, functional and hormone markers were determined by standard laboratory methods using certified assays in the local clinical laboratory (United Laboratories of Tartu University Hospital, Estonia). After the 1-week probiotic or placebo consumption the values of hematological and inflammatory indices, metabolic, functionality markers stayed within the normal range in all volunteers. Some mild changes after PM II consumption occurred (**Table 10**), but did not exceed the normal references values in any of those blood markers values.

**Table 10.** Effect of one week consumption of probiotics containing novel strains or placebo on blood hematological and inflammatory indices, metabolic, functionality and hormone markers in volunteers

| | PMI (n=15) | | | PM II (n=14) | | | Placebo (n=28) | | | Reference interval |
|---|---|---|---|---|---|---|---|---|---|---|
| | Before | After | *P* | Before | After | *P* | Before | After | *P* | |
| WBC (E9/L) | 6.0 ± 1.3 | 5.8 ± 1.4 | 0.254 | 6.5 ± 2.2 | 7.0 ± 1.7 | 0.208 | 6.2 ± 1.6 | 6.2 ± 1.7 | 0.569 | 3.5 to 8.8 |
| RBC (E12/L) | 4.5 ± 0.3 | 4.5 ± 0.3 | 0.908 | 4.7 ± 0.4 | 4.7 ± 0.4 | 0.527 | 4.5 ± 0.3 | 4.5 ± 0.3 | **0.051** | 3.9 to 5.2 |
| Hb (g/L) | 130.5 ± 6.4 | 130.5 ± 7.6 | 0.977 | 131.6 ± 10.7 | 13.4 ± 13.0 | 0.185 | 131.6 ± 11.8 | 129.5 ± 10.6 | 0.157 | 117.0 to 153.0 |
| Hct(%) | 39.4 ± 2.1 | 39.5 ± 1.9 | 0.907 | 39.3 ± 2.7 | 40.0 ± 3.3 | 0.585 | 39.7 ± 3.0 | 39.9 ± 2.6 | **0.036** | 35.0 to 46.0 |
| Ph (E9/L) | 255.7 ± 59.6 | 293.2 ± 68.7 | 0.977 | 288.7 ± 82.6 | 299.2 ± 72.4 | 0.220 | 295.3 ± 73.7 | 237.4 ± 70.5 | 0.592 | 145.0 to 390.0 |
| Eo E9/L | 0.1 ± 0.1 | 0.2 ± 0.2 | 0.097 | 0.2 ± 0.1 | 0.2 ± 0.2 | 0.405 | 0.2 ± 0.1 | 0.2 ± 0.1 | 0.185 | < 0.5 |
| Baso (E9/L) | 0.0 ± 0.0 | 0.0 ± 0.0 | 1.000 | 0.0 = 0.0 | 0.0 ± 0.0 | 1.000 | 0.0 ± 0.0 | 0.0 ± 0.0 | 1.000 | <0.1 |
| Mono (E9/L) | 0.5 ± 0.1 | 0.5 ± 0.2 | 0.231 | 0.6 ± 0.2 | 0.6 ± 0.2 | 0.825 | 0.6 ± 02 | 0.6 ± 0.2 | 0.711 | 0.2 to 1.0 |
| Lymph (E9/L) | 2.4 ± 0.6 | 2.4 ± 0.6 | 0.908 | 2.8 ± 1.3 | 2.9 ± 0.9 | 0.489 | 2.5 ± 07 | 2.6 ± 0.8 | 0.846 | 1.0 to 3.0 |
| Neut (E9/L) | 3.0 ± 0.9 | 2.8 ± 0.8 | 0.201 | 2.8 ± 1.0 | 3.2 ± 1.1 | 0.157 | 2.9 ± 1.0 | 2.9 ± 0.9 | 0.927 | 2.0 to 7.0 |
| CRP-hs (mg/L) | 1.2 ± 1.3 | 1.0 ± 1.2 | 0.531 | 1.1 ± 1.7 | 1.0 ± 1.0 | 0.974 | 1.0 ± 0.9 | 1.0 ± 0.8 | 0.305 | > 5.0 |
| Gluc (mmol/L) | 4.9 ± 0.6 | 4.8 ± 1.2 | 0.305 | 4.9 ± 0.3 | 5.0 ± 0.3 | **0.028** | 4.9 ± 0.6 | 4.9 ± 0.6 | 0.963 | 4.5 to 6.0 |
| ALAT (U/L) | 14.8 ± 7.8 | 14.9 ± 7.1 | 0.374 | 16.1 ± 13.9 | 15.3 ± 7.1 | 0.392 | 15.5 ± 6.1 | 14.1 ± 5.7 | 0.155 | < 33.0 |
| ASAT (U/L) | 19.7 ± 8.3 | 13.3 ± 6.2 | 0.276 | 19.2 ± 6.6 | 19.2 ± 4.1 | 0.614 | 20.0 ± 5.3 | 18.6 ± 4.9 | 0.136 | < 32.0 |
| GGT (U/L) | 12.5 ± 5.3 | 12.7 ± 5.5 | 0.562 | 14.8 ± 10.8 | 13.9 ± 6.0 | 0.307 | 13.2 ± 5.2 | 13.1 ± 5.4 | 0.342 | < 40.0 |
| Crea (μmol/L) | 68.3 ± 8.4 | 67.0 ± 7.0 | 0.232 | 68.2 ± 7.5 | 72.1 ± 10.6 | **0.038** | 69.8 ± 8.6 | 70.3 ± 12.1 | 0.972 | 44.0 to 80.0 |
| Chol (mmol/L) | 4.5 ± 0.6 | 4.7 ± 0.8 | 0.172 | 4.7 ± 0.7 | 4.8 ± 0.4 | 0.314 | 4.9 ± 0.6 | 4.7 ± 0.7 | 0.129 | 2.9 to 6.1 |
| LDL (mmol/L) | 2.5 ± 0.5 | 2.7 ± 0.6 | 0.317 | 2.7 ± 0.6 | 2.8 ± 0.4 | 0.592 | 2.7 ± 0.6 | 2.7 ± 0.6 | 0.639 | 1.2 to 4.3 |
| Prog (pmol/L) | 7.8 ± 10.0 | 11.4 ± 17.7 | 0.306 | 19.2 ± 26.4 | 11.3 ± 21.8 | 0.777 | 10.2 ± 17.9 | 9.0 ± 15.5 | 0.673 | - |

(continued)

| | PMI (n=15) | | | PM II (n=14) | | | Placebo (n=28) | | | Reference interval |
|---|---|---|---|---|---|---|---|---|---|---|
| | Before | After | P | Before | After | P | Before | After | P | |
| E2 (pmol/L) | 282.7 ± 273.4 | 379.0 ± 502.1 | 0.789 | 379.6 ±335.5 | 401.9 ± 545.6 | 0.729 | 432.3 ± 474.2 | 345.6 ± 379.2 | 0.459 | - |

WBC = white blood cells; RBC =red blood cells; Hb = hemoglobin; Hct = hematocrit; Plt = platelets; Eo = eosinophils; Baso = basophils; Mono = monocytes; Lymph = lymphocytes; Neut = neutrophils; CRP-hs = C-reactive protein high-sensitive; Gluc = glucose; ALAT = alanine aminotransferase; ASAT = aspartate aminotransferase; GGT = gamma-glutamyl transferase, Creat = creatinine; Chol = cholesterol; LDL= low-density lipoprotein.

*Impact of probiotic consumption on vaginal and intestinal microbial communities*

The vaginal and stool microorganisms were quantified using real-time PCR (qPCR) method, including total bacterial count and counts of lactobacilli, *L. crisptatus, G. vaginalis, A. vaginae* and *C. albicans* (**Table 11**). Most of the changes were statistically non-significant, however, we noted reduction in *G. vaginalis* count in PM II group ($\log_{10}$ 3.57 *vs* 2.38; p= 0.027) and reduction of total bacterial count in PM I group ($\log_{10}$ 7.99 *vs* 7.72; p= 0.048). Some changes were just on significance level like increase in total bacterial count and total lactobacilli count in PM II group. The proportion of *L. crispatus* in total bacterial counts increased after the probiotic consumption.

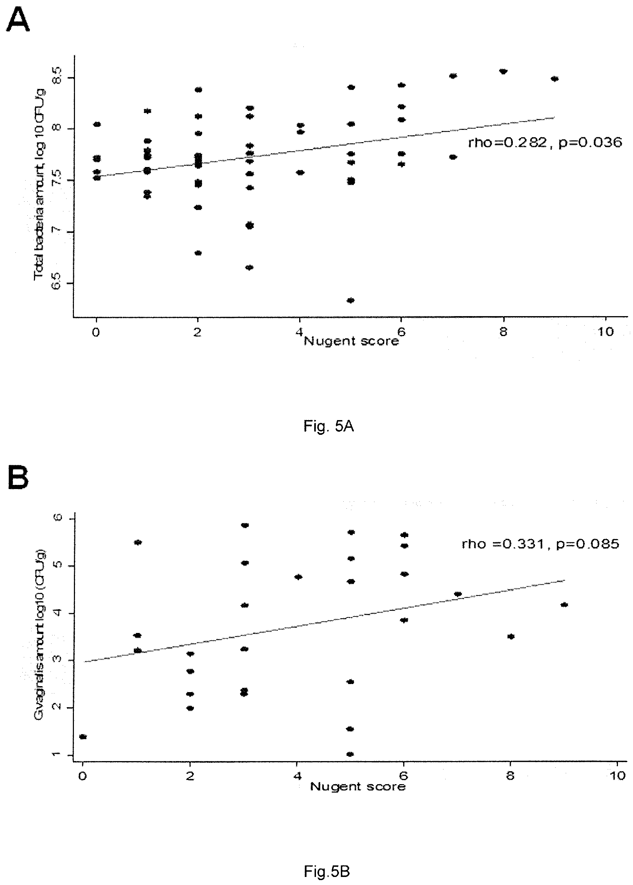

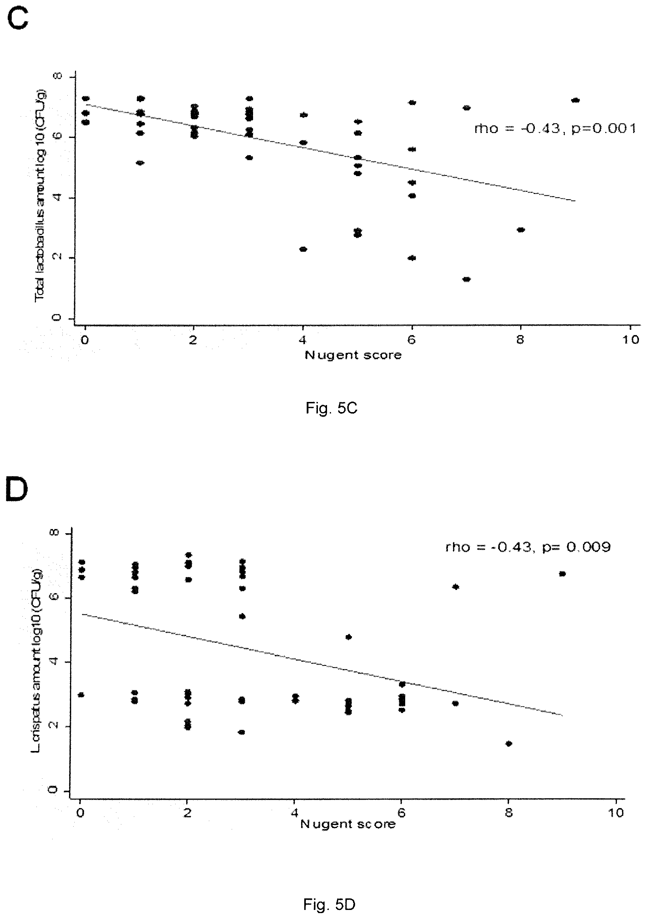

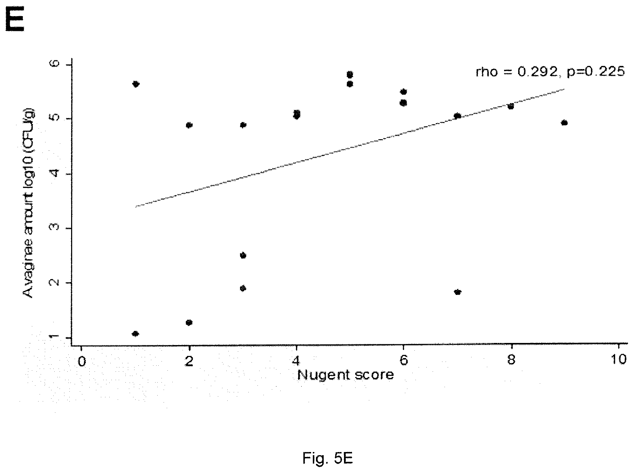

The vaginal microbiota status was additionally evaluated by using Nugent scoring. Presence of clue cells, white blood cells and yeasts was recorded as well. The PM II intake resulted in a significant decrease in Nugent score between the treatment period visits from median 3.0 to 2.0 (mean 3.9 to 2.6, p=0.002) while the change was insignificant in case of PM I and placebo consumers (**Figure 4**). We revealed negative correlations between Nugent score and total lactobacillus counts and *L. crispatus* counts while positive correlations between Nugent score and total bacterial counts and counts of *G. vaginalis* and *A. vaginae* (**Figure 5**). No correlation between the counts of fecal lactobacilli and Nugent scores was found.

**Table 11.** Quantification of *L. crispatus, G. vaginalis, A. vaginae and C. albicans* from the vaginal samples (calculated as median and IQR)

| | Vaginal samples log$_{10}$(CFU/g) | | | | | | | | | | | |
| | *Lactobacillus crispatus* | | | *Gardnerella vaginalis*** | | | *Atopobium vaginae**** | | | *Candida albicans* **** | | |
| | At the beginning | At the end | *p** | At the beginning | At the end | *p** | At the beginning | At the end | *p** | At the beginning | At the end | *p** |
|---|---|---|---|---|---|---|---|---|---|---|---|---|
| PM I | 338 (2.94-634) | 2.97 (2.78-6.34) | 0.826 | 4.35 (2.61-5.56) | 3.53 (2.55-5.66) | 0.345 | 4.84 (3.96-4.96) | 5.20 (5.04-5.29) | 0.686 | 2.16 (1.69-4.34) | 0.00 (0-1.55) | 0.109 |
| PM II | 3.05 (2.87-6.70) | 4.65 (2.76-6.81) | 0.925 | 3.57 (2.38-4.69) | 2.38 (1.55-3.14) | 0.027 | 4.14 (3.02-4.53) | 4.88 (1.89-5.82) | 0.593 | 1.94 (1.17-2.70) | 2.00 (0.00-4.00) | 0.655 |
| Placebo | 2.98 (2.83-6.03) | 2.90 (2.72-6.68) | 0.525 | 3.95 (2.56-5.23) | 4.41 (3.85-4.83) | 0.382 | 3.54 (1.82-4.89) | 5.03 (2.50-5.48) | 0.314 | 1.11 (0.96-2.10) | 0.46 (0-1.85) | 0.068 |

* Wilcoxon paired signed-rank test
** Positive samples: PM I (n=9) PM II (n=9) Placebo (n=20);
*** Positive samples: PM I (n=5) PM II (n=5) Placebo (n=11);
**** Positive samples: PM I (n= 3) PM II (n=2) Placebo (n= 6).

EP 3 636 782 A1

**Example 13 Clinical trial in vaginitis patients**

*Study information*

182 antibiotic-naive vaginitis patients of reproductive age (18-50 year old) were recruited into the trial. Absence of chronic diseases was confirmed by questionnaire and blood analysis at screening visit. Study participants consumed 1 capsule per day during 3 months (20 capsules per month, during intermenstrual periods), and every participant had monthly trial visits.

During the first visit, the patients gave informed consent and filled questionnaire about general and gynaecological health and lifestyle. During the first and last visit, gynaecologist performed gynaecological exams and collected blood samples. Vaginal samples were collected monthly and were delivered to the laboratory immediately, where they were subsequently frozen at -80 °C. During the study period, patients completed short weekly questionnaire about general feeling, gynaecological and gastrointestinal health.

89 of the patients presented with bacterial vaginosis (BV). Of them, 25 women dropped out for various reasons (antibiotic treatment, pregnancy, positive STD test, no wish to continue, too low Nugent score). In a randomized double-blind placebo-controlled parallel-arm study, the eligible participants were randomly allocated to one of three groups and had to consume either (i) the oral study product, an oral capsule containing probiotic mixture (strains DSM32720 and DSM32717, in total $3\times10^{10}$ CFU); or (ii) the vaginal study product, a vaginal capsule containing probiotic mixture (the same strains, in total $3\times10^{10}$ CFU); or (iii) oral placebo capsules.

93 of the patients presented with vulvovaginal candidiasis (VVC). Of them, 30 women dropped out for various reasons (antibiotic treatment, pregnancy, positive STD test, no wish to continue). In a randomized double-blind placebo-controlled parallel-arm study, the eligible participants were randomly allocated to one of three groups and had to consume either (i) the oral study product, an oral capsule containing probiotic mixture (strains DSM32720, DSM32718 and DSM32716, in total $3\times10^{10}$ CFU); or (ii) the vaginal study product, a vaginal capsule containing probiotic mixture (the same strains, in total $3\times10^{10}$ CFU); or (iii) oral placebo capsules.

*Vaginal health monitoring*

Assessment of vaginal health was via weekly questionnaire and monthly vaginal swab. Vaginal discharge questions were based on markers about discharge amount, consistency, color, odor and itchiness. We hereby present the results of first month of the trial based on Nugent score in BV patients and combined score of two important complaints (amount of discharge and itchiness) in WC patients. Nugent score decreased both in case of oral and vaginal capsules while the change was non-significant in case of placebo capsules. The combined score in VVC patients decreased both in case of oral and vaginal capsules while the change was non-significant in case of placebo capsules (Table 12).

**Table 12.** Change of Nugent score in BV patients and combined score in VVC patients after consuming study products during one month.

| Weeks | Patients with BV (mean ± SD) | | |
|---|---|---|---|
| | Oral capsules * | Vaginal capsules * | Placebo capsules |
| | (n=23) | (n=22) | (n=19) |
| W1 | 7.6 ± 1.8 | 7.6 ± 1.4 | 7.1 ± 2.0 |
| W4 | 4.4 ± 3.0 | 4.4 ± 2.5 | 7,0 ± 1.8 |
| | Patients with WC (mean ± SD) | | |
| | Oral capsules * (n=19) | Vaginal capsules * (n=17) | Placebo capsules (n=27) |
| W 1 | 4.3 ± 2.2 | 3.1 ± 1.8 | 3.9 ± 2.1 |
| W 2 | 3.9 ± 1.9 | 4.1 ± 2.0 | 3.4 ± 2.1 |
| W 3 | 2.9 ± 1.9 | 2.2 ± 1.6 | 3.0 ± 2.2 |
| W 4 | 2.4 ± 1.9 | 2.1 ± 1.9 | 2.9 ± 2.2 |
| * P<0.05 (Kruskal-Wallis test) | | | |

**Claims**

1. A Lactobacillus crispatus strain having the identifying characteristics of the strain deposited under Accession Number DSM 32717, DSM 32715, DSM 32716, DSM 32718, DSM 32719, or DSM 32720; or a mutant or variant of any thereof.

2. A Lactobacillus crispatus mutant or variant according to claim 1, wherein said mutant or variant substantially retains at least following characteristics of the wild-type strain:

   - the ability to produce lactic acid;
   - the ability to produce $H_2O_2$;
   - antagonistic activity against E. coli ATCC 700414, Candida albicans ATCC 32032, and Gardnerella vaginalis ATCC 14018 (DSM 4944),

   wherein said mutant or variant is optionally **characterised in that** it contains a recombinant gene or vector.

3. A composition comprising, consisting essentially, or consisting of exactly or at least 1, 2, 3, 4, 5 or 6 different Lactobacillus crispatus strains selected from the strains defined in claim 1.

4. A composition according to claim 3, wherein the composition comprises, consists essentially, or consists of at least Lactobacillus crispatus strain DSM 32717, DSM 32718, or DSM 32720, or a combination of any 2 or all 3 thereof.

5. The composition according to claim 4, wherein the composition comprises:

   i) Lactobacillus crispatus strain DSM 32720; or
   ii) Lactobacillus crispatus strain DSM 32717 and DSM 32720; or
   iii) i) Lactobacillus crispatus strain DSM 32716, DSM 32718 and DSM 32720.

6. A composition according to any one of claims 3 to 5, wherein the composition further comprises (i) one or more mutants or variants according to claim 2 and/or
   (ii) one or more further probiotic bacteria, e.g. selected from *Lactobacillus acidophilus, L. brevis, L. bulgaricus, L. casei, L. delbrueckii, L. divergens, L. fermentum, L. gasseri, L. helveticus, L. jensenii, L. johnsonii, L. kunkeei, L. lactis, L. otakiensis, L. paracasei, L. plantarum, L. reuteri, L. rhamnosus, L. ruminis, L. sakei, L. salivarius, L. vaginalis, Lactococcus diacetylactis, Lactococcus lactis, Lactococcus thermophilus, Bifidobacterium bifidum, Bifidobacterium lactis, Bifidobacterium longum, Enterococcus faecium* and/or *Streptococcus thermophilus.*

7. A composition according to any one of claims 3 to 6, wherein the composition further comprises one or more further components, e.g. selected from (a) one or more source(s) of nutrients, e.g. a prebiotic; (b) one or more mineral salt(s); (c) one or more cryoprotectants; (d) one or more pH stabilizing agent(s); one or more agents with antimicrobial properties.

8. A composition according to any one of claims 3 to 7, wherein the composition is a pharmaceutical composition and further comprises a pharmaceutically acceptable carrier, diluent and/or excipient.

9. A composition according to any one of claims 3 to 7, wherein the composition further comprises one or more nutrients and is a nutraceutical composition or a food product.

10. A hygiene product, e.g. selected from as a sanitary napkin, diaper, panty liner, tampon, incontinence guard, and/or hygiene tissue, said product comprising one or more Lactobacillus crispatus strains according to claim 1, mutant or variant according to claims 1 or 2 and/or a composition according to any one of claims 3 to 8.

11. A Lactobacillus crispatus strain according to claim 1, mutant or variant according to claims 1 or 2 and/or a composition according to any one of claims 3 to 8 for use in therapy, preferably the treatment or prevention of dysbiosis, vaginosis, vulval and/or vaginal yeast infection, villitis, vaginitis and/or urinary tract infection.

12. A method of treating or preventing a condition selected from dysbiosis, vaginosis, vulval and/or vaginal yeast infection, villitis, vaginitis and/or urinary tract infection, said method comprising administering to a subject in need thereof an effective amount of a Lactobacillus crispatus strain according to claim 1, mutant or variant according to claims 1 or 2 and/or a composition according to any one of claims 3 to 8.

13. A product containing at least one Lactobacillus crispatus strain according to claim 1 and at least one further probiotic bacterium as a combined preparation for the simultaneous, separate or sequential use in therapy, preferably the treatment or prevention of dysbiosis, vaginosis, vulval and/or vaginal yeast infection, villitis, vaginitis and/or urinary tract infection.

14. A Lactobacillus crispatus strain according to claim 1, mutant or variant according to claims 1 or 2 and/or a composition according to any one of claims 3 to 8 for use in modulating the microbiota of a subject, e.g. restoring a beneficial vaginal microbiota.

15. A method of modulating the microbiome, e.g. vaginal microbiome, of a subject, said method comprising administering to a subject in need thereof an effective amount of a Lactobacillus crispatus strain according to claim 1, mutant or variant according to claims 1 or 2 and/or a composition according to any one of claims 3 to 8.

16. A product containing at least one Lactobacillus crispatus strain according to claim 1 and at least one further probiotic bacterium as a combined preparation for the simultaneous, separate or sequential use in modulating the microbiome, e.g. vaginal microbiome, of a subject.

Fig.1

Fig.2

Fig.3

Fig. 4

Fig. 5A

Fig.5B

C

Fig. 5C

D

Fig. 5D

Fig. 5E

| DSM 32715 | DSM 32720 | DSM 32719 | DSM 32718 | DSM 32717 | DSM 32716 | Strain/substrate | DSM 32715 | DSM 32720 | DSM 32719 | DSM 32718 | DSM 32717 | DSM 32716 | Strain/substrate |
|---|---|---|---|---|---|---|---|---|---|---|---|---|---|
| o | o | o | o | o | o | Esculin ferric citrate | o | o | o | o | o | o | Glycerol |
| o | o | o | 1 | o | o | Salicin | o | o | o | o | o | o | Erythritol |
| o | o | o | o | o | o | D-Cellobiose | o | o | o | o | o | o | D-Arabinose |
| 1 | 1 | 1 | 1 | 1 | 1 | D-Maltose | o | o | o | o | o | o | L-Arabinose |
| 1 | 1 | 1 | 1 | 1 | 1 | D-Lactose (bovine origin) | o | o | o | o | o | o | D-Ribose |
| o | o | o | o | o | o | D-Melibiose | o | o | o | o | o | o | D-Xylose |
| 1 | 1 | 1 | 1 | 1 | 1 | D-Saccharose (sucrose) | o | o | o | o | o | o | L-Xylose |
| o | o | o | o | o | o | D-Trehalose | o | o | o | o | o | o | D-Adonitol |
| o | o | o | o | o | o | Inulin | o | o | o | o | o | o | Methyl-βD-Xylopyranoside |
| o | o | o | o | o | o | D-Melezitose | 1 | 1 | 1 | 1 | 1 | 1 | D-Galactose |
| o | o | o | o | o | o | D-Raffinose | 1 | 1 | 1 | 1 | 1 | 1 | D-Glucose |
| 0.5 | 0.5 | 1 | o | 1 | 1 | Amidon (starh) | 1 | 1 | 1 | 1 | 1 | 1 | D-Fructose |
| o | o | o | o | o | 0.5 | Glycogen | 1 | 1 | 1 | 1 | 1 | 1 | D-Mannose |
| o | o | o | o | o | o | Xylitol | o | o | o | o | o | o | L-Sorbose |
| o | o | o | o | o | o | Gentiobiose | o | o | o | o | o | o | L-Rhamnose |
| o | o | o | o | o | o | D-Turanose | o | o | o | o | o | o | Dulcitol |
| o | o | o | o | o | o | D-Lyxose | o | o | o | o | o | o | Inositol |
| o | o | o | o | o | o | D-Tagatose | 0.5 | 0.5 | 0.5 | 0.5 | o | o | D-Mannitol |
| o | o | o | o | o | o | D-Fucose | o | o | o | o | o | o | D-Sorbitol |
| o | o | o | o | o | o | L-Fucose | o | o | o | o | o | o | Methyl-αD-Mannopyranoside |
| o | o | o | o | o | o | D-Arabitol | o | o | o | o | o | o | Methyl-αD-Glycopyranoside |
| o | o | o | o | o | o | L-Arabitol | 0.5 | 0.5 | o | o | 1 | 0.5 | N-Acetylglucosamine |
| o | o | o | o | o | o | potassium Gluconate | o | o | o | o | o | o | Amygdalin |
| o | o | o | o | o | o | potassium 2-ketogluconate | o | o | o | o | o | o | Arbutin |
| o | o | o | o | o | o | potassium 5-ketogluconate | | | | | | | |

Fig. 6

Europäisches
Patentamt

European
Patent Office

Office européen
des brevets

**EUROPEAN SEARCH REPORT**

Application Number

EP 19 20 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/021733 A1 (ABRAMOV VYACHESLAV MIHAYLOVICH [RU] ET AL.) 6 February 2014 (2014-02-06) * See Engl. Abstract; Tables 6, 7 (p.21-22) See also D2 (Abramov et al. 2014) for scientific disclosure in English. * ----- | 1-16 | INV. C12R1/225 A61K35/747 |
| X | VYACHESLAV ABRAMOV ET AL: "Probiotic Properties of Lactobacillus crispatus 2,029: Homeostatic Interaction with Cervicovaginal Epithelial Cells and Antagonistic Activity to Genitourinary Pathogens", PROBIOTICS AND ANTIMICROBIAL PROTEINS, vol. 6, no. 3-4, 16 July 2014 (2014-07-16) , pages 165-176, XP055647056, New York, NY ; Heidelberg : Springer ISSN: 1867-1306, DOI: 10.1007/s12602-014-9164-4 * Whole doc., in partic. Abstract; Tables 1, 2, 4-6 * ----- | 1-16 | |
| X | WO 2016/026474 A1 (VS CHEMICKO TECHNOLOGICKA V PRAZE [CZ]; ARKO CONSULT S R O [CZ]) 25 February 2016 (2016-02-25) * Whole doc., in partic. p.11-13. Table VIII * ----- | 1-16 | TECHNICAL FIELDS SEARCHED (IPC) C12R A61K |
| X | WO 98/46261 A1 (GYNELOGIX INC [US]) 22 October 1998 (1998-10-22) * Whole doc., in partic. p.31, p.67 (example 11) * ----- | 1-16 | |
| | -/-- | | |

The present search report has been drawn up for all claims

| Place of search | Date of completion of the search | Examiner |
|---|---|---|
| Munich | 7 February 2020 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 20 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. E. STAPLETON ET AL: "Randomized, Placebo-Controlled Phase 2 Trial of a Lactobacillus crispatus Probiotic Given Intravaginally for Prevention of Recurrent Urinary Tract Infection", CLINICAL INFECTIOUS DISEASES, vol. 52, no. 10, 14 April 2011 (2011-04-14), pages 1212-1217, XP055275134, US ISSN: 1058-4838, DOI: 10.1093/cid/cir183 * e.g. p.1216, col.2 * | 1-16 | |
| X | US 2015/190438 A1 (NIVOLIEZ ADRIEN [FR]) 9 July 2015 (2015-07-09) * Whole doc., in partic. para. [0065,0080]; Tables 5, 6A, 6B * | 1-16 | |
| X | US 2010/151026 A1 (LIU YANG [US] ET AL) 17 June 2010 (2010-06-17) * Whole doc., in partic. para. [0016,0046]; Methods, section 3 * | 1-16 | TECHNICAL FIELDS SEARCHED (IPC) |
| X | WO 2018/013583 A2 (THE BRIGHAM AND WOMEN'S HOSPITAL INC [US]) 18 January 2018 (2018-01-18) * Whole doc., in partic. para. [0006,00037,000197]; Fig.22A * | 1-16 | |
| X,P | M. ROSTOK ET AL: "Potential vaginal probiotics: safety, tolerability and preliminary effectiveness", BENEFICIAL MICROBES, vol. 10, no. 4, 19 April 2019 (2019-04-19), pages 385-393, XP055647090, NL ISSN: 1876-2883, DOI: 10.3920/BM2016.0123 * Applicants disclosure of strains of invention. Whole doc. * | 1-16 | |

The present search report has been drawn up for all claims

| Place of search | Date of completion of the search | Examiner |
|---|---|---|
| Munich | 7 February 2020 | Roscoe, Richard |

## ANNEX TO THE EUROPEAN SEARCH REPORT
## ON EUROPEAN PATENT APPLICATION NO.

EP 19 20 2134

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2020

| Patent document cited in search report | | Publication date | Patent family member(s) | | Publication date |
|---|---|---|---|---|---|
| WO 2014021733 | A1 | 06-02-2014 | NONE | | |
| WO 2016026474 | A1 | 25-02-2016 | NONE | | |
| WO 9846261 | A1 | 22-10-1998 | AT | 400281 T | 15-07-2008 |
| | | | AU | 732630 B2 | 26-04-2001 |
| | | | BR | 9808520 A | 15-01-2002 |
| | | | CA | 2285947 A1 | 22-10-1998 |
| | | | CN | 1259874 A | 12-07-2000 |
| | | | EP | 1011721 A1 | 28-06-2000 |
| | | | JP | 2001523231 A | 20-11-2001 |
| | | | JP | 2010031031 A | 12-02-2010 |
| | | | KR | 20010006273 A | 26-01-2001 |
| | | | NZ | 500788 A | 29-04-2003 |
| | | | NZ | 513835 A | 28-09-2001 |
| | | | US | 6093394 A | 25-07-2000 |
| | | | US | 6372209 B1 | 16-04-2002 |
| | | | US | 2002090365 A1 | 11-07-2002 |
| | | | WO | 9846261 A1 | 22-10-1998 |
| US 2015190438 | A1 | 09-07-2015 | AU | 2013289309 A1 | 29-01-2015 |
| | | | BR | 112014033026 A2 | 27-06-2017 |
| | | | CA | 2878130 A1 | 16-01-2014 |
| | | | CN | 104508117 A | 08-04-2015 |
| | | | EP | 2870234 A1 | 13-05-2015 |
| | | | ES | 2647833 T3 | 26-12-2017 |
| | | | FR | 2992973 A1 | 10-01-2014 |
| | | | HK | 1207116 A1 | 22-01-2016 |
| | | | KR | 20150036376 A | 07-04-2015 |
| | | | PT | 2870234 T | 30-11-2017 |
| | | | RU | 2015103116 A | 27-08-2016 |
| | | | TW | 201408773 A | 01-03-2014 |
| | | | US | 2015190438 A1 | 09-07-2015 |
| | | | WO | 2014009330 A1 | 16-01-2014 |
| US 2010151026 | A1 | 17-06-2010 | NONE | | |
| WO 2018013583 | A2 | 18-01-2018 | US | 2019307817 A1 | 10-10-2019 |
| | | | WO | 2018013583 A2 | 18-01-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NUGENT et al.** Reliability of diagnosing bacterial vaginosis is improved by a standardized method of gram stain interpretation. *Journal of Clinical Microbiology,* 1991, vol. 29 (2), 297-301
- **ESCHENBACH et al.** Prevalence of hydrogen peroxide-producing Lactobacillus species in normal women and women with bacterial vaginosis. *J Clin Microbiol,* 1989, vol. 27 (2), 251-256
- **HOLDEMAN et al.** Anaerobe Laboratory Manual. Virginia Polytechnic Institute and State Laboratory, 1977
- Algorithm for Identification of Aerobic Gram-Positive Cocci. **PFALLER et al.** Manual of Clinical Microbiology. American Society for Microbiology, 2007
- **KOS et al.** Adhesion and aggregation ability of probiotic strain Lactobacillus acidophilus M92. *J Appl Microbiol,* 2003, vol. 94 (6), 981-987
- **BUJNAKOVA ; KMET.** Aggregation of animal lactobacilli with 0157 enterohemorrhagic Escherichia coli. *J Vet Med B Infect Dis Vet Public Health,* 2002, vol. 49 (3), 152-154
- **KAEWNOPPARAT et al.** In vitro probiotic properties of Lactobacillus fermentum SK5 isolated from vagina of a healthy woman. *Anaerobe,* 2013, vol. 22, 6-13

- **CLERMONT et al.** New tetracycline resistance determinants coding for ribosomal protection in streptococci and nucleotide sequence of tet(T) isolated from Streptococcus pyogenes A498. *Antimicrob Agents Chemother,* 1997, vol. 41 (1), 112-116
- **JACOBSEN et al.** Screening of probiotic activities of forty-seven strains of Lactobacillus spp. by in vitro techniques and evaluation of the colonization ability of five selected strains in humans. *Appl Environ Microbiol,* 1999, vol. 65 (11), 4949-4956
- **STRUS et al.** The in vitro activity of vaginal Lactobacillus with probiotic properties against Candida. *Infect Dis Obstet Gynecol,* 2005, vol. 13 (2), 69-75
- **SIMPSON et al.** Genomic diversity and relatedness of bifidobacteria isolated from a porcine cecum. *J Bacteriol,* 2003, vol. 185, 2571-81
- **SCHAAB et al.** Analysis of High Accuracy, Quantitative Proteomics Data in the MaxQB Database. *Mol Cell Proteomics,* 2012
- **KOENIG et al.** Robust prediction of the MASCOT score for an improved quality assessment in mass spectrometric proteomics. *J. Proteome Res.,* 2008, vol. 7 (9), 3708-17